# EUROPEAN PATENT SPECIFICATION

(11) **EP 4 293 359 B1**
(45) Date of publication and mention of the grant of the patent: **10.06.2026**
(21) Application number: 22179079.3
(22) Date of filing: 15.06.2022
(51) Int. Cl.: G01N 33/68, A61P 1/02

(54) **SULCUS-PROTEINS FOR THE DETECTION OF PERI-IMPLANTITIS**
SULCUS-PROTEINE ZUR DETEKTION VON PERIIMPLANTITIS
PROTÉINES DE SULCUS POUR LA DÉTECTION DE LA PÉRI-IMPLANTITE

(43) Date of publication of application: 20.12.2023
(73) Proprietor: Albert-Ludwigs-Universität Freiburg Körperschaft des Öffentlichen Rechts, 79098 Freiburg (DE)
(72) Inventor: Nelson, Katja, 79100 Freiburg im Breisgau (DE); Fretwurst, Tobias, 79104 Freiburg im Breisgau (DE); Schilling, Oliver, CH4052 Basel (CH); Halstenbach, Tim, 79106 Freiburg im Breisgau (DE); Iglhaut, Gerhard, 97700 Memmingen (DE)
(74) Representative: Krauss, Jan

(56) References cited:
- EP-A2- 2 583 684
- WO-A1-2015/117881
- HENTENAAR DIEDERIK F. M. ET AL: "Biomarker levels in peri-implant crevicular fluid of healthy implants, untreated and non-surgically treated implants with peri-implantitis", JOURNAL OF CLINICAL PERIODONTOLOGY, vol. 48, no. 4, 16 February 2021 (2021-02-16), DK, pages 590 - 601, XP055977213, ISSN: 0303-6979, Retrieved from the Internet <URL:https://onlinelibrary.wiley.com/doi/full-xml/10.1111/jcpe.13423> DOI: 10.1111/jcpe.13423
- G�NC� G�LIZ N. ET AL: "Effect of inflammation on cytokine levels and bone remodelling markers in peri-implant sulcus fluid: A preliminary report", CYTOKINE, vol. 59, no. 2, 1 August 2012 (2012-08-01), US, pages 313 - 316, XP055977268, ISSN: 1043-4666, DOI: 10.1016/j.cyto.2012.04.024
- ANONYMOUS: "Gene Family: Immunoglobulin heavy locus at 14q32.33 (IGH)", HGNC, 31 July 2017 (2017-07-31), XP055408985, Retrieved from the Internet <URL:http://www.genenames.org/cgi-bin/genefamilies/set/349> [retrieved on 20170922]

## Description

The present invention relates to a method, in particular an *in vitro* method, for a method for detecting peri-implantitis (PI) in a mammalian subject having a tooth implant, comprising detecting, and quantifying at least one protein in a sulcus fluid sample obtained from said subject that is enriched or depleted in the sample when compared to a sulcus fluid sample obtained from a healthy implant (I) and/or a healthy tooth (T), wherein an enrichment of at least one protein as identified and/or wherein a depletion of at least one protein as identified detects a PI in said subject. Furthermore, the present invention relates to the application of the findings of the invention in the development of new anti-PI therapies and as well as to a kit for performing the above methods as well as respective uses thereof. Finally, improved anti-PI compounds or pharmaceutical compositions are provided.

### Background of the invention

Worldwide, annually about 12 million tooth implants are inserted, and about 1 million in Germany (Albrektsson 2014, VDDl 2015). Long-term survival rates of dental implants are documented over 90% after ten years. However, peri-implant inflammation (peri-implant mucositis: PM; peri-implantitis: PI) is common with a prevalence of up to 20% for peri-implantitis and up to 40% for peri-implant mucositis (Derks et al. 2016). The etiology is assumed to be determined by a microbial biofilm in a susceptible host. Prosthetic, surgical or biomechanical factors are discussed as an additional etiological mechanism for peri-implant bone loss (Canullo et al. 2015; Albrektsson et al. 2016; Fretwurst et al. 2018). 50 % of the implants show disease within 3 years after insertion and show a progredient course of disease which can lead to a loss of the implant (Derks 2016). Currently no standardized therapy of diseased implants is available, the treatment options are associated with high recurrence rates even after a decade of intensive research (Esposito et al. 2012; Carcuac et al. 2020). Drug therapies with a curative approach are not available (Ramanauskaite et al. 2021).

Early non-invasive diagnostic tools or parameters are missing, only invasive, unspecific diagnostic tools e.g. peri-implant pocket depth measurement, radiological peri-implant bone level are available to detect peri-implantitis (Schwarz 2016).

Radiological bone loss and probing depth indicate peri-implantitis when the disease has already progressed. The microbiological analysis of peri-implant disease has not demonstrated any consistent, disease-specific microbiome profiles and can therefore not be used for diagnosis or disease monitoring (Lafaurie et al. 2017; Sahrmann et al. 2020).

In the field of periodontology and implantology, potential diagnostic markers of periodontitis in the gingival crevicular fluid (GCF) and for peri-implantitis in the peri-implant crevicular fluid (PICF) are discussed as a point-of-care testing laboratory diagnostics (Sorsa et al. 2017). Potential diagnostic, prognostic or therapeutic markers include enzymes, inflammation, and host reaction mediators e.g., cytokines, interleukins as well as metabolic markers related to bone tissue degeneration around the tooth or the implant. For the detection of the markers targeted methods e.g. ELISA, spectrophotometry or flow cytometry were mainly used (Duarte et al. 2016; Arias-Bujanda et al. 2019; Kensara et al. 2021; Iglhaut et al. 2021). In the targeted approach potential diagnostic markers must be selected a priori, and only a limited number of markers can be evaluated. Due to the limitation of the analytical methods used only a small number of proteins have been evaluated, the proteomic composition of the sulcus fluid has not been evaluated to date.

Immunohistologic examination of inflamed peri-implant und periodontal tissues demonstrated larger areas of inflammatory infiltrates in peri-implantitis compared to periodontitis. Furthermore, macrophages (CD-68 positive cells), plasma cells (CD-138 positive cells) and neutrophils (myeloperoxidase (MPO) -positive cells) are detected in the peri-implant infiltrates (Carcuac und Berglundh 2014). Inflamed peri-implant tissue demonstrates a pro-inflammatory M1 macrophage polarization and a lymphocyte-dominated inflammation with interindividual, patient-specific differences (Fretwurst et al. 2016; Fretwurst et al. 2021; Fretwurst et al. 2020). The concept of patient-specific immune profiles in peri-implantitis was corroborated by transcriptome sequencing of the peri-implant inflammatory tissue and has been proposed to be regarded for risk stratification for the therapeutic success of surgical peri-implant therapy (Wang et al. 2021).

KR20220022835A discloses a biomarker for diagnosing peri-implantitis, a composition for diagnosing peri-implantitis using the same, and a diagnostic kit using the same, and more particularly, to a biomarker for diagnosing peri-implantitis using an oral microbiome, a composition for diagnosing peri-implantitis using the same, and to a diagnostic kit as used.

de Aguiar MC, et al. (in: The Gingival Crevicular Fluid as a Source of Biomarkers to Enhance Efficiency of Orthodontic and Functional Treatment of Growing Patients. Biomed Res Int. 2017;2017:3257235. doi: 10.1155/2017/3257235. Epub 2017 Jan 23. PMID: 28232938; PMCID: PMC5292379) review that gingival crevicular fluid (GCF) is a biological exudate and quantification of its constituents is a current method to identify specific biomarkers with reasonable sensitivity for several biological events. Studies are being performed to evaluate whether the GCF biomarkers in growing subjects reflect both the stages of individual skeletal maturation and the local tissue remodeling triggered by orthodontic force. Present evidence is still little regarding whether and which GCF biomarkers are correlated with the growth phase (mainly pubertal growth spurt), while huge investigations have been reported on several GCF biomarkers (for inflammation, tissue damage, bone deposition and resorption, and other biological processes) in relation to the orthodontic tooth movement. In spite of these investigations, the clinical applicability of the method is still limited with further data needed to reach a full diagnostic utility of specific GCF biomarkers in orthodontics.

Hentenaar DFM, et al. (in: Biomarker levels in peri-implant crevicular fluid of healthy implants, untreated and non-surgically treated implants with peri-implantitis. J Clin Periodontol. 2021 Apr;48(4):590-601. doi: 10.1111/jcpe.13423. Epub 2021 Feb 16. PMID: 33454996) discloses a method for detecting peri-implantitis (Pl) in a mammalian subject having a tooth implant, comprising detecting, and quantifying at least one protein in a su|cus fluid sample obtained from said subject, wherein an enrichment of at least one protein selected from the group consisting of IL-1 beta and MMP8 detects a PI in said subject.

Güncü GN, et al. (in: Effect of inflammation on cytokine levels and bone remodelling markers in peri-implant sulcus fluid: a preliminary report. Cytokine. 2012 Aug;59(2):313-6. doi: 10.1016/j.cyto.2012.04.024. Epub 2012 May 14. PMID: 22592038) diasclose a method for detecting peri-implantitis (Pl) in a mammalian subject having a tooth implant (abstract, here: dental implant), comprising detecting, and quantifying at least one protein in a sulcus fluid sample obtained from said subject that is enriched in the sample, wherein an enrichment of at least one protein selected from the group consisting of IL-1 beta, IL-10, and OPG detects a PI in said subject.

WO 2015/117881 A1 relates to a method for determining the state of peri-implant disease comprising the steps of quantifying the expression level of one or more regulated markers of a group of markers forming a panel, said one or more regulated markers being related to the plasminogen system and/or inflammation and/or proteolytic activity or combinations thereof or ratios thereof in an ex vivo sample; and determining the state of peri-implant disease by comparing the expression level obtained in step a with a reference value.

In personalized cancer precision medicine, molecular information/profiling of tumors, the microenvironment and the diseased individual is increasingly used for diagnostics and treatment decisions (Le Tourneau et al. 2019; Ahadi et al. 2020). Mass spectrometry (MS) based proteomics enables quantification and identification of almost all proteins in the medium allowing for multibiomarker approaches and non-targeted exploratory essays (Kwon et al. 2016; Fretwurst 2021, under revision). In the field of periodontology, targeted proteomic methods enabled the evaluation of approximately 100 proteins (Loos und Tjoa 2005), when using MS based proteomics more than 600 proteins were identified in the gingival crevicular fluid of periodontitis diseased teeth (Rizal et al. 2020). Studies demonstrated increased expressions of apolipoproteins, immunoglobulins as well as cytoskeletal proteins and histones of neutrophil extracellular traps (NETs) (Bostanci et al. 2010; Nguyen et al. 2020). The PICF proteome has not been systematically investigated by comparing healthy and diseased implants nor has the proteomic composition found in PICF been compared to the composition of GCF (Esberg et al. 2019). MS-based proteomics of PICF has only been performed once, not including healthy implants or healthy teeth as a control group (Esberg et al. 2019), therefore not allowing for the identification of disease specific markers or proteomic clusters.

There is therefore a marked interest to provide non-invasive methods for a detection of periimplantitis in contrast to healthy non-inflamed implants. It is therefore an object of the present invention to provide respective assays and methods that reliably and conveniently identify periimplantitis. Other objects and advantages will become apparent for the person of skill from studying the present description including the examples and Figures.

In a first aspect thereof, the present invention solves the above problem by providing a method for detecting peri-implantitis (PI) in a mammalian subject having a tooth implant, comprising detecting, and quantifying at least one protein in a sulcus fluid sample obtained from said subject that is enriched or depleted in the sample when compared to a sulcus fluid sample i) obtained from a healthy implant (I), ii) a healthy tooth (T), iii) an earlier sulcus fluid sample taken from said subject, and/or iv) to a control sample, wherein an enrichment of the protein IGHV70D, optionally together with an enrichment of at least one protein selected from the group consisting of, ARPC5, XRP2, DYSF, FLOT1, APOM, HGPRT, CA2, HBD, LRG, PRBP, CAT, CD18, SERPING1, MPO, HC-II, PYGL, GPI, BPGM, ANX6, RNASE2, ITGAM, G6PD, ANX3, ALADH, NQO2, BPI, ITGB5, AZU1, GGH, LTA4H, MIG9, HMG-2, STOM, GCA, TKT, DDT, PRDX3, SRI, CORO1A, LSP-1, CHI3L1, TALDO1, MNDA, CAMP, ARHGDIB, HK3, HBA1, BASP1, BST1, MTO, FLOT2, PLBD1, CNN2, SEC11A, GAPR-1, ADSF, APMAP, and PADI2 and/or optionally together with a depletion of at least one protein selected from the group consisting of COPE, NARS1, ATP5MG, EMAP-2, HLA-A, CTSD, ANX2, CAPNS1, CTSB, HSP90AA1, TACSTD2, TXN, COX4I1, PLS3, EZR, CBR1, RPL7, PEBP-1, S100A11, MDH1, ALDH9A1, NAPA, SLC25A3, PSMD2, PCN, TKFC, NAP-2, ACO2, ENOPH1, JPT1, and PSME2 detects a PI in said subject..

Preferred is a method for detecting peri-implantitis (PI) in a mammalian subject having a tooth implant according to the present invention, wherein the at least one protein in the sulcus fluid sample is secreted or shedded, such as, for example, a protein selected from the group consisting of FLOT1, APOM, CD18, MPO, ITGAM, BPI, ITGB5, FLOT2, PLBD1, ADSF, and APMAP, and/or CTSD.

In a second aspect thereof, the present invention solves the above problem by providing a method for diagnosing the status of peri-implantitis (PI) in a mammalian subject having a tooth implant, comprising performing the method according to the present invention, and diagnosing an exacerbated state of the PI if the at least one protein in the sulcus fluid sample obtained from said subject is further enriched or further depleted in the sample when compared to an earlier sulcus fluid sample obtained from the subject.

In a third aspect thereof, the present invention solves the above problem by providing a method for identifying a compound that is active against PI, comprising the steps of performing the method according to the present invention on a sulcus fluid sample obtained from a subject suffering from PI that has been administered with a candidate compound, wherein a lesser enrichment and/or a lesser depletion of at least one protein in the sulcus fluid sample in the presence of said candidate compound, when compared to the absence of said candidate compound identifies a compound that is active against PI.

In a fourth aspect thereof, the present invention solves the above problem by providing a method for monitoring of a treatment or prophylaxis against PI in a mammalian subject in need thereof, comprising a) performing the method according to the present invention on a biological sample obtained from a subject that has been administered with a compound or pharmaceutical composition that is active against PI, and b) comparing the enrichment and/or depletion of at least one protein in the sulcus fluid sample as detected with the enrichment and/or depletion of the at least one protein in an earlier sulcus fluid sample taken from said subject, and/or to a control sample.

It was surprisingly found in the context of the present invention that healthy implants (I) and healthy teeth (TI) exhibit a markedly different, significant and specific sulcus protein signature than diseased implants with peri-implantitis (PI). Thus, proteomics enables identification of specific proteins in the peri-implant crevicular fluid (PICF), also termed herein peri-implant sulcus fluid or sulcus fluid. In the context of the present invention, the sulcus fluid is of particular interest, and in the context of the present invention "sulcus fluid" shall mean the fluid in the crevices between tooth or implant, respectively, and the gingival tissue. Sulcus has to be distinguished from sputum. Sulcus fluid is usually and preferably collected from the sulcus in a minimum depth of 1-2 mm and may be collected with sterile paper strips by insertion for about 30 sec, like, for example, gingival crevicular fluid (GCF). Preferred examples are endodontic paper points or periopapers.

Nazar Majeed, Zeyad et al. (in: Identification of Gingival Crevicular Fluid Sampling, Analytical Methods, and Oral Biomarkers for the Diagnosis and Monitoring of Periodontal Diseases: A Systematic Review. Disease markers vol. 2016 (2016): 1804727. doi:10.1155/2016/1804727) evaluate from current scientific literature the most common and precise method for gingival crevicular fluid (GCF) sample collection, biomarker analytical methods, and the variability of biomarker quantification, even when using the same analytical technique. 73.1% of the researchers analyzed their samples by using enzyme-linked immunosorbent assay (ELISA). 22.6%, 19.5%, and 18.5% of the researchers included interleukin-1 beta (IL-1*β*), matrix metalloproteinase-8 (MMP-8), and tumor necrosis factor-alpha (TNF-α), respectively, in their studies as biomarkers for periodontal disease. Paper strips are the most convenient and accurate method for gingival crevicular fluid collection, while enzyme-linked immunosorbent assay can be considered the most conventional method for the diagnosis of biofluids.

In recent years, some biomarkers in the peri-implant sulcus fluid (PICF) were mainly examined using ELISA, spectrophotometry, or flow cytometry (Alassy et al. 2019). Nevertheless, using these analytical techniques only a very limited number of proteins could be detected, and the functional composition of the sulcus fluid could not be sufficiently assessed.

Pro-inflammatory cytokines, such as TNF, IL-1, IL-6, IL-12, IL-17, anti-inflammatory cytokines, such as IL-4 and IL-10, biomarkers of bone metabolism, such as receptor activator nuclear factor kappa-B ligand (RANKL), receptor activator nuclear factor B (RANK), osteoprotegerine (OPG) and osteocalcin, as well as specific enzymes, such as matrix metalloproteinases (MMP1, MMP8, MMP9, MMP13) and tissue inhibitor of metalloproteinases (TIMP) were in the focus of periimplantitis research (Alassy et al. 2019, Iglhaut et al. 2021).

Hu Z, et al. (in: Inflammatory cytokine profiles in the crevicular fluid around clinically healthy dental implants compared to the healthy contralateral side during the early stages of implant function. Arch Oral Biol. 2019 Dec;108:104509. doi: 10.1016/j.archoralbio.2019.104509. Epub 2019 Jul 29. PMID: 31494437) describe and compare cytokines levels in clinically healthy sites of dental implants and natural teeth.

Thus, research focused on only some biomarkers that were selected *a priori,* and so far have not been validated for a diagnosis.

As mentioned above, in a first aspect thereof, the present invention solves the above problem by providing a method for detecting peri-implantitis (PI) in a mammalian subject having a tooth implant, comprising detecting, and quantifying at least one protein in a sulcus fluid sample obtained from said subject that is enriched or depleted in the sample when compared to a sulcus fluid sample i) obtained from a healthy implant (I), ii) a healthy tooth (T), iii) an earlier sulcus fluid sample taken from said subject, and/or iv) to a control sample, wherein an enrichment of the protein IGHV70D, optionally together with an enrichment of at least one protein selected from the group consisting of, ARPC5, XRP2, DYSF, FLOT1, APOM, HGPRT, CA2, HBD, LRG, PRBP, CAT, CD18, SERPING1, MPO, HC-II, PYGL, GPI, BPGM, ANX6, RNASE2, ITGAM, G6PD, ANX3, ALADH, NQO2, BPI, ITGB5, AZU1, GGH, LTA4H, MIG9, HMG-2, STOM, GCA, TKT, DDT, PRDX3, SRI, CORO1A, LSP-1, CHI3L1, TALDO1, MNDA, CAMP, ARHGDIB, HK3, HBA1, BASP1, BST1, MTO, FLOT2, PLBD1, CNN2, SEC11A, GAPR-1, ADSF, APMAP, and PADI2 and/or optionally together with a depletion of at least one protein selected from the group consisting of COPE, NARS1, ATP5MG, EMAP-2, HLA-A, CTSD, ANX2, CAPNS1, CTSB, HSP90AA1, TACSTD2, TXN, COX4I1, PLS3, EZR, CBR1, RPL7, PEBP-1, S100A11, MDH1, ALDH9A1, NAPA, SLC25A3, PSMD2, PCN, TKFC, NAP-2, ACO2, ENOPH1, JPT1, and PSME2 detects a PI in said subject.

With a lack of specific detection and diagnostic tools for peri-implantitis and only minor understanding of underlying molecular biology, diagnostics, therapy, and prognostics of peri-implant diseases pose a challenge to clinicians (Ramanauskaite et al. 2021). The present invention thus assessed the proteomic composition of PICF of peri-implantitis affected implants in comparison to healthy implants and healthy teeth in an untargeted approach to reveal the proteomic difference between healthy and diseased implants. Furthermore included is a method that detects PI based on the changes when compared to an earlier sulcus fluid sample taken from said subject, i.e. based on the "internal" comparison of markers.

PLS-DA and *LIMMA* as performed in the context of the present invention indicated no alterations in the proteomic composition of PICF/GCF of healthy teeth (T) and healthy implants (I). In contrast, 59 proteins were identified as upregulated and 31 downregulated in peri-implantitis when compared to healthy implants.

In the context of the present invention, peri-implantitis (PI) is defined according to the current international guideline with peri-implant pocket depth ≥ 6 mm, ≥ 3 mm peri-implant radiological bone loss with bleeding and / or suppuration on probing (Berglundh 2018).

It was furthermore surprisingly found in the context of the present invention, that classical secreted or shedded proteins from the cell surface are found in a significantly higher number in the enriched proteins, when compared with the depleted proteins. In view of this, and also in view of the more convenient collect and analysis of these proteins (in particular the shedded parts thereof), preferred is the method for detecting peri-implantitis (PI) in a mammalian subject having a tooth implant according to the present invention, wherein the at least one protein in the sulcus fluid sample is secreted or shedded, such as, for example, a protein selected from the group consisting of FLOT1, APOM, CD18, MPO, ITGAM, BPI, ITGB5, FLOT2, PLBD1, ADSF, APMAP, and CTSD.

Shedded proteins as herein relates to the proteolytic removal of proteins from the surface of a cell, in particular of membrane protein ectodomains (ectodomain shedding) which designates a post-translational modification that controls levels and function of hundreds of membrane proteins. The contributing proteases are referred to as sheddases.

Secreted proteins as herein relates to proteins or protein fragments released from a cell. Secretion of proteins usually is a multistep "active" cellular process that involves vesicle biogenesis, cargo loading, concentration and processing, vesicle transport and targeting, vesicle docking and Ca2+-dependent vesicular fusion with the plasma membrane.

Both shedded proteins (or the fragments thereof) and secreted proteins are thus found in the cell-free part (e.g., the supernatant) of the sulcus fluid sample, which is a preferred embodiment of the sample to be used in the context of the present invention.

As mentioned above, it was surprisingly found in the context of the present invention that healthy implants (I) and healthy teeth (TI) exhibit a significantly different and specific sulcus protein signature than diseased implants with peri-implantitis (PI). Therefore, preferred is the method for detecting peri-implantitis (PI) in a mammalian subject having a tooth implant according to the present invention, wherein the significance of the at least one protein has a moderated p-value of < 0.05, more preferably of < 0.01. Further preferred is the method for detecting peri-implantitis (PI) in a mammalian subject having a tooth implant according to the present invention, wherein the significance of the combination of the proteins as analyzed has a moderated p-value of < 0.05, more preferably of < 0.01, and most preferred of less than 0.005.

Sulcus fluid is usually and preferably collected from the sulcus in a minimum depth of 1-2 mm and may be collected with sterile paper strips by insertion for about 30 sec, like, for example, gingival crevicular fluid (GCF). Preferred examples are endodontic paper points or periopapers. Preferred is the method for detecting peri-implantitis (PI) in a mammalian subject having a tooth implant according to the present invention, wherein the sulcus fluid sample was obtained from said subject using sterile paper strips such as, for example, are endodontic paper points or periopapers.

In general, the detection and quantification of the at least one protein in the sample, in particular the secreted and/or shedded proteins or fragments thereof can be performed by any suitable method as is known to the person of skill. Preferred is the method for detecting peri-implantitis (PI) in a mammalian subject having a tooth implant according to the present invention, wherein the detection and quantification of the at least one protein in the sample comprises a method selected from at least one of Enzyme-linked Immunosorbent Assay (ELISA), proteolysis, bicinchoninic acid assay, and mass spectrometry. These methods and assays are disclosed herein and known to the person of skill.

In another aspect of the present invention, a higher proteolytic activity in PI was found in peri-implantitis, with cleavage patterns pointing to typical inflammatory proteases cathepsin G and neutrophil elastase. In inflammatory diseases, endogenous proteolysis inherits an important role, which has not been assessed for peri-implantitis previously. Cathepsin G and neutrophil elastase were identified in all samples with higher levels in the diseased condition, although without significance. Therefore, preferred is the method for detecting peri-implantitis (PI) in a mammalian subject having a tooth implant according to the present invention, further comprising detecting endogenous proteolysis in the sample, wherein a higher endogenous proteolytic activity as detected when compared to a non-PI sample, a T sample (sample from a healthy tooth), an I sample (sample from a healthy implant) and/or a control sample indicates a PI-sample (sample from a subject suffering from PI) from a subject.

In yet another aspect of the present invention, the inventors studied endogenous proteolysis in peri-implantitis, and performed an additional data analysis step with a spectral library that also represents N- or C-terminally truncated peptides of the human proteome. Peptides were identified which were of semi-specific nature, and increased proportional intensities of semi-specific peptides were found in PI with statistical significance compared to healthy teeth (p = 0.0057), indicating that peri-implantitis lesions possess higher endogenous proteolytic activity. Therefore, preferred is the method for detecting peri-implantitis (PI) in a mammalian subject having a tooth implant according to the present invention, wherein detecting a higher endogenous proteolytic activity comprises detecting of proportional intensities of semi-specific peptides.

In yet another aspect of the present invention, the inventors performed a mass spectrometry-based assessment of bacterial proteins in PICF, demonstrating that the relative intensity of bacterial proteins was increased in peri-implantitis. Nevertheless, it is in question whether bacterial proteins can serve as diagnostic indicators of PI (Mombelli und Décaillet 2011; Wang et al. 2016). Therefore, preferred is the method for detecting peri-implantitis (PI) in a mammalian subject having a tooth implant according to the present invention, further comprising detecting and quantifying bacterial proteins in the sample, wherein a higher amount as detected when compared to a non-PI sample, a T sample, an I sample and/or a control sample indicates a PI sample. This analysis further supports the results as obtained with the sulcus-proteins as above.

Also, missingness of bacterial proteins was high in all conditions, with almost no proteins found in more than 50% of samples of I and T, indicating a disparate and inhomogeneous bacterial distribution in peri-implantitis. Again, this analysis further supports the results as obtained with the sulcus-proteins as above. Thus, preferred is the method for detecting peri-implantitis (PI) in a mammalian subject having a tooth implant according to the present invention, further comprising detecting the missingness of bacterial proteins in the sample, wherein a lower missingness as detected when compared to a non-PI sample, a T sample, an I sample and/or a control sample indicates a PI sample.

The present method may be performed with a sample from any mammal having a tooth implant, suspected of suffering from PI. Preferred is the method for detecting peri-implantitis (PI) in a mammalian subject having a tooth implant according to the present invention, wherein the mammalian subject is selected from a cat, dog, mouse, rat, horse, sheep, goat, monkey, cow, or human. Preferred is a human.

Another aspect of the present invention then relates to a method for diagnosing the status of peri-implantitis (PI) in a mammalian subject having a tooth implant, comprising performing the method according to the present invention, and diagnosing an exacerbated state of the PI if the at least one protein in the sulcus fluid sample obtained from said subject is further enriched or further depleted in the sample when compared to an earlier sulcus fluid sample obtained from the subject.

In another aspect of the present invention, the present invention can be used to improve the prevention and treatment of a PI in a subject. The present invention provides a method for identifying a compound that is active against PI, comprising the steps of performing the method according to the present invention on a sulcus fluid sample obtained from a subject suffering from PI that has been administered with a candidate compound, wherein a lesser enrichment and/or a lesser depletion of at least one protein in the sulcus fluid sample in the presence of said candidate compound, when compared to the absence of said candidate compound identifies a compound that is active against PI. In addition, the compound may have an effect on the bacterial proteins as identified and/or the missingness thereof.

In principle, any suitable candidate compound may be screened. Preferred is the method for identifying a compound that is active against PI according to the present invention, wherein the candidate compound is selected from the group consisting of a chemical molecule, a molecule selected from a library of small organic molecules (molecular weight less than 500 Da), a molecule selected from a combinatory library, a cell extract, in particular a plant cell extract, a small molecular drug, a protein, a protein fragment, a molecule selected from a peptide library, and an antibody or fragment thereof. Methods for screening are known in the art, preferred is a high-throughput method (HTS). Preferred is a screening in a suitable animal model. These candidate molecules may also be used as a basis to screen for improved compounds. Thus, preferred is the method according to the present invention, wherein after the identification of the anti-PI compound, the method further comprises the step of chemically modifying the compound. In general, many methods of how to modify compounds of the present invention are known to the person of skill and are disclosed in the literature. Modifications of the compounds will usually fall into several categories, for example a) mutations/changes of amino acids into different amino acids, b) chemical modifications, e.g., through the addition of additional chemical groups, c) changes of the size/length of the compound, and/or d) the attachment of additional groups to the molecule (including marker groups, labels, linkers or carriers, such as chelators). In a next step, the modified compound is tested again in at least one of tests as above, and if the property of the compound is improved compared to its unaltered state.

Pharmaceutical compositions as used may optionally comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO2), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for the compounds as herein and will readily be able to choose suitable pharmaceutically acceptable carriers or excipients, depending, e.g., on the formulation and administration route of the pharmaceutical composition.

The pharmaceutical composition can be administered orally, e.g., in the form of pills, tablets, coated tablets, sugar coated tablets, hard and soft gelatin capsules, solutions, syrups, emulsions or suspensions or as aerosol mixtures. Administration, however, can also be carried out rectally, e.g., in the form of suppositories, or parenterally, e.g., in the form of injections or infusions, or percutaneously, e.g. in the form of ointments, creams or tinctures.

The pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain the aforementioned salts of two or more compounds as identified according to the invention and also other therapeutically active substances as described herein.

Yet another aspect of the present invention then relates to a method, such as diagnostic method, for monitoring of a treatment or prophylaxis against PI in a mammalian subject in need thereof, comprising a) performing the method according to the present invention on a biological sample obtained from a subject that has been administered with a compound or pharmaceutical composition that is active against PI, and b) comparing the enrichment and/or depletion of at least one protein in the sulcus fluid sample as detected with the enrichment and/or depletion of the at least one protein in an earlier sulcus fluid sample taken from said subject, and/or to a control sample. Preferred is the method, wherein a decrease of the enriched protein and/or an increase of the depleted protein is indicative for the success of, progress of and/or sensitivity for the anti-IP treatment or prophylaxis in the mammalian subject. The method may further comprise adjusting the treatment or prophylaxis of said subject or patient based on said monitoring. The attending physician will be readily able to make and apply respective treatment decisions, also taking into account additional patient parameters, if required.

As mentioned herein, the compound is administered to said subject in an effective dosage. This dosage can vary within wide limits and is to be suited to the individual conditions in each individual case. For the above uses, the appropriate dosage will vary depending on the mode of administration, the particular condition to be treated and the effect desired. In general, however, satisfactory results are achieved at dosage rates are as above, e.g. of about 1 to 100 mg/kg animal body weight particularly 1 to 50 mg/kg. Suitable dosage rates for larger mammals, for example humans, are of the order of from about 10 mg to 3 g/day, conveniently administered once or in divided doses, e.g. 2 to 4 times a day, or in sustained release form. In general, a daily dose of approximately 10 mg to 100 mg, particularly 10 to 50 mg, per human individual is appropriate in the case of the oral administration. An effective concentration to be reached at the cellular level can be set at between 50 to 200 µM, preferably at about 100 µM. Particularly preferred is topical application, such as to the airways by inhalation. In these cases, the dosage can be conveniently reduced to between 0.1 to 10 mg/dose, preferably 0.2 to 5 mg per dose, which equals about 3 to about 80 µg per kilogram for a 70 kg subject.

It is to be understood that the present compound and/or a pharmaceutical composition comprising the compound is for use to be administered to a human patient. The term "administering" means administration of a sole therapeutic agent or in combination with another therapeutic agent. It is thus envisaged that the pharmaceutical composition of the present invention is employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the compound for use, if required, as long as they act in combination (i.e. directly and/or indirectly, preferably synergistically) with the present compound for use.

Thus, the compounds can be used alone or in combination with other active compounds - for example with medicaments already known for the prevention or treatment of PI, whereby in the latter case a favorable additive, amplifying or preferably synergistically effect is noticed. Suitable amounts to be administered to humans range from 1 to 500 mg, in particular 5 mg to 100 mg, such as between 1 and 10 mg/kg/day oral dose. An effective concentration to be reached at the cellular level can be set at between 50 to 200 µM, preferably at about 100 µM.

In the medical use aspects, the compound (for use) can be provided and/or is administered as a suitable pharmaceutical composition, such as a tablet, capsule, injection, granule, powder, sachet, reconstitutable powder, dry powder inhaler, inhalation, and/or chewable. Such solid formulations may comprise excipients and other ingredients in suitable amounts. Such solid formulations may contain e.g., cellulose, cellulose microcrystalline, polyvidone, in particular FB polyvidone, magnesium stearate and the like. Administration, however, can also be carried out rectally, e.g. in the form of suppositories, or parenterally, e.g. in the form of injections or infusions, or percutaneously, e.g. in the form of ointments, creams or tinctures.

Preferred is the use of the (local) inhibition of proteases as identified in the sulcus; further preferred examples include the injection of inhibitor depots.

The present invention is based on the findings by the inventors, when sulcus samples were analyzed in a retrospective cohort study using quantitative proteomics (liquid chromatography-tandem spectrometry, LC-MS/MS). A depletion of highly abundant proteins was not necessary. The initial study included 11 cases of healthy implants and 18 cases of peri-implantitis. Using linear models of microarray analysis (limma), the markers according to the present invention were found which were significantly (pa_{djusted} < 0.05; mean quantitative difference > 50%) enriched or depleted in the sulcus of the peri-implantitis (compared to healthy implants): The significant enriched or depleted proteins are listed in the following two tables. Sulcus also contains cellular debris or cellular components. Classical secreted or shed proteins from the cell surface are marked. It is interesting to note that in peri-implantitis significantly more classically secreted or shed of the relevant proteins are enriched than depleted.

The following uses can be derived from the present invention:
Peri-implantitis (PI) can be detected in a non-invasive, biochemical way by detecting and quantifying the inventive sulcus proteins (alone or in combination). Classically secreted or shed proteins are particularly suitable for detecting peri-implantitis.

The significant accumulation of secreted proteolytic enzymes, such as MMP-9 (with gelatinolytic and collagenolytic activity) and cathepsin G opens up new perspectives for curbing tissue-degrading processes through (local) inhibition of these proteases; examples include the injection of inhibitor depots.

The present invention should form the basis for the translational application and further development of the inventors' findings in order to enable longitudinal, multi-modal monitoring of peri-implant diseases in the near future and, based on this, to pursue personalized prevention and therapy concepts (Steinhubl 2019, Schüssler -Fiorenza Rose 2019).

With a lack of specific diagnostic tools for peri-implantitis and only minor understanding of underlying molecular biology, diagnostics, therapy, and prognostics of peri-implant diseases pose a challenge to clinicians (Ramanauskaite et al. 2021). The present invention thus first assessed the proteomic composition of PICF of peri-implantitis affected implants in comparison to healthy implants and healthy teeth in an untargeted approach to reveal the proteomic difference between healthy and diseased implants.

Over 2300 different proteins were identified and quantified in this study, exceeding previous proteomic studies on crevicular fluid, which were based on data dependent acquisition mass spectrometry. In the earlier data dependent acquisition-based studies, a maximum of 600 proteins were identified in GCF (Tsuchida et al. 2013; Rizal et al. 2020; Nguyen et al. 2020) and for PICF, the only existing study identified up to 200 proteins (Esberg et al. 2019). Data-independent acquisition (DIA) was applied for quantitative proteomics of crevicular fluid in the present study. DIA has proven to produce more robust results, less vulnerable to stochastic biases, undersampling or limited reproducibility than conventional data-dependent acquisition (DDA) (Bruderer et al. 2015; Gillet et al. 2012; Kelstrup et al. 2018). The results as obtained in the context of the present invention show that DIA-approaches in combination with the here presented protein extraction protocol may enable deeper proteome decryption, especially when dealing with small sample volume as in crevicular fluid (Ludwig et al. 2018; Marinho et al. 2019).

PLS-DA and *LIMMA* indicated no alterations in the proteomic composition of PICF/GCF of healthy teeth and healthy implants. In contrast, 90 proteins were identified as either upregulated (enriched) or downregulated (depleted) in peri-implantitis when compared to healthy implants. To date, no study has been published comparing the proteomic crevicular fluid composition of healthy and diseased implants, nor of healthy implants and healthy teeth. Esberg et al. found 42 proteins to be increased in case of implant loss after peri-implantitis therapy in comparison to peri-implantitis therapy success (Esberg et al. 2019). Several studies have described altered protein expressions between healthy and periodontally diseased teeth and indicated a discrimination of periodontally healthy and diseased teeth on the proteome level (Shin et al. 2019; Marinho et al. 2019; Bostanci et al. 2010). Accordingly, the PLS-DA-results of the present invention revealed a difference between peri-implantitis and healthy implants as well as healthy teeth.

In the present invention, gene ontology analysis revealed biological processes related to local immune response like "humoral immune response", "extracellular matrix organization" or "regulated exocytosis" to be upregulated in peri-implantitis. This is in accordance with the classic depiction of peri-implantitis as a local inflammation, orchestrated by the host immune response (Berglundh et al. 2018). Cytoskeletal proteins like fibrinogens, actins, or actin related proteins were found upregulated in the present invention as well as in previous publications on periodontitis, suggesting an increase of apoptosis (Bostanci et al. 2015; Rizal et al. 2020). Proteomic studies of GCF and samples of gingival tissue revealed elevated levels of immunoglobulins in periodontitis, therefore emphasizing the humoral response (Baliban et al. 2012; Marinho et al. 2019; Bao et al. 2020). Interestingly, only few complement components (C9 and C8 gamma chain) were found elevated the present invention (p_{mod} < 0.05, not significant after multiple testing correction), while no immunoglobulins were detected as differentially expressed in PI. Hence, humoral response may play a minor role in peri-implantitis than in periodontitis. Proinflammatory cytokines, as discussed by a variety of publications using antibody-based protein detection in periodontitis and peri-implantitis, were not identified in the present invention. Missingness of cytokines in MS-based proteomic studies has frequently been attributed to low concentrations of the molecules of interest (Baliban et al. 2012; Bostanci et al. 2010). For quantification of cytokines, targeted MS-based approaches are preferably applied.

Several proteins expressed by neutrophiles (myeloperoxidase, bacterial permeability-increasing protein, azurocidin, cathelicidin antimicrobial peptide), inheriting antibacterial activity, were identified in higher abundance in the peri-implantitis proteome. Azurodicin is expressed by neutrophiles and shows antimicrobial activity against Gram-negative species, including *P. aeruginosa* and *A. actinomycetemcomitans,* by binding lipopolysaccharides (LPS) (Wasiluk et al. 1991; Campanelli et al. 1990). Likewise, myeloperoxidase is expressed in neutrophiles and acts synergistically with azurocidin (Furtmüller et al. 1998). In periodontology, azurodicin and myeloperoxidase were found to be elevated in GCF of periodontitis sites, using both ELISA and MS-based proteomics (Nalmpantis et al. 2020; Rizal et al. 2020; Bostanci et al. 2015). Also, myeloperoxidase was found more abundant in PICF of implants with peri-implantitis (Durrani und Singh 2015), which is in accordance with the findings of the present invention.

Higher proteolytic activity in PI was found in peri-implantitis, with cleavage patterns pointing to typical inflammatory proteases cathepsin G and neutrophil elastase. In inflammatory diseases, endogenous proteolysis inherits an important role, which has not been assessed for peri-implantitis previously. Cathepsin G and neutrophil elastase were identified in all samples with higher levels in the diseased condition, although without significance. Both proteases are secreted by neutrophiles in azurophilic granules alongside neutrophilic proteins (Korkmaz et al. 2010). Neutrophile granulocytes have been found in peri-implantitis lesions both in higher proportion and in higher total amounts when compared to periodontitis (Carcuac und Berglundh 2014). Increased proteolytic activity by cathepsin G and/or neutrophil elastase as well as upregulation of azurocidin and myeloperoxidase, as presented here, underlines elevated neutrophile activity in peri-implant inflammation.

In the present invention, proteins involved in inflammatory processes were detected to be enriched e.g. S100P, leukotriene A(4) hydrolase (LTA4H) or lymphocyte specific protein 1 (LSP-1), which have not been described in crevicular fluid before (Rizal et al. 2020; Salazar et al. 2013; Esberg et al. 2019). LSP-1 and LTA4H inherit proinflammatory properties through their contribution to migration and lamellipodia formation of macrophages (Maxeiner et al. 2015; Schäringer et al. 2021) and conversion of leukotriene A4 into proinflammatory leukotriene B4 (Tholander et al. 2008), respectively. S100P is a calcium binding protein localized in the cytoplasm and the nucleus. Calcium-binding of S100P leads to activation of several target proteins, such as RAGE (receptor for advanced glycation end points) (Jiang et al. 2012). RAGE is strongly associated with inflammation and immunologic diseases (Palanissami und Paul 2018) and, among other effects, may activate nuclear factor-κB (NF-κB) (Arumugam et al. 2004), which is connected to bone homeostasis via receptor activator of NF-κB (RANK) and RANK-Ligand (RANKL) (Krakauer 2008; Khosla 2001). The latter were shown to be elevated in PICF of peri-implantitis, which accords with increased osteoclast activity of crestal bone around diseased implants (Rakic et al. 2013; Andrucioli et al. 2019; Gürlek et al. 2017). S100P may play a central role in inflammation and bone homeostasis and resemble a link between immune response and crestal bone loss (Palanissami und Paul 2018). This is the first time to find elevated levels of S100P in case of PI.

Interestingly, other members of the S100-family (S100A6, S100A7, S100A8, S100A9) have frequently been found upregulated in GCF of periodontally diseased teeth (Rizal et al. 2020). Besides S100P, no significantly elevated levels of calcium-binding S100-proteins were detected. In contrast S100A11 was significantly decreased in PI whereas in periodontitis S100A11 was found elevated in GCF (Kido et al. 2012). These differences regarding S100-proteins may indicate distinct differences of the crevicular fluid composition between periodontitis and peri-implantitis on the proteome level.

Matrix metalloproteinases (MMPs) 8 and 9 were identified in all samples and most abundant in the diseased subgroup (PI), yet without significance. MMPs have frequently been observed to be elevated at periodontally diseased sites, with some publications also describing upregulation in case of peri-implantitis (Gianazza et al. 2016; Sorsa et al. 2016; Alassiri et al. 2018; Alassy et al. 2019; Arakawa et al. 2012). Previous studies mainly focused on MMP8, a collagenase, secreted by neutrophils (Arakawa et al. 2012; Basegmez et al. 2012; Alassy et al. 2019). No MMP dominated fingerprint of endogenous proteolysis was detected in the present invention.

In the present invention, mass spectrometry-based assessment of bacterial proteins in PICF was performed for the first time, demonstrating that the relative intensity of bacterial proteins was increased in peri-implantitis. Elevated proportions of bacterial proteins were also found by Bostanci et al. (2010) for periodontitis. Missingness of bacterial proteins was high in all conditions, with almost no proteins found in more than 50% of samples of I and T, indicating a disparate and inhomogeneous bacterial distribution in peri-implantitis. This is in accordance with microbiome studies, pointing to a diverse and ambiguous microbiomes associated with peri-implantitis (Mombelli und Décaillet 2011; Persson und Renvert 2014; Renvert et al. 2007; Sahrmann et al. 2020). Therefore, it is in question whether bacterial proteins can serve as diagnostic indicators of PI (Mombelli und Décaillet 2011; Wang et al. 2016).

The present invention demonstrates the potency of mass spectrometry-based proteomics on crevicular fluid of peri-implantitis. Ideally, because of the small cohort size larger cohorts would provide an even better correlation of the proteomic data with patient individual clinical data to enable evaluations of potential etiological pathways. The PICF proteome of peri-implantitis affected sites exhibits an inflammatory fingerprint when compared to healthy implants and teeth. Healthy implants and teeth share strong similarities in their proteome. The method according to the present invention will pave the way to a more profound understanding of disease biology, new diagnostic and prognostic tools and a more patient-specific therapy of peri-implantitis in the future.

The invention will now be further described in the following examples and with reference to the accompanying figures, without being limited thereto.

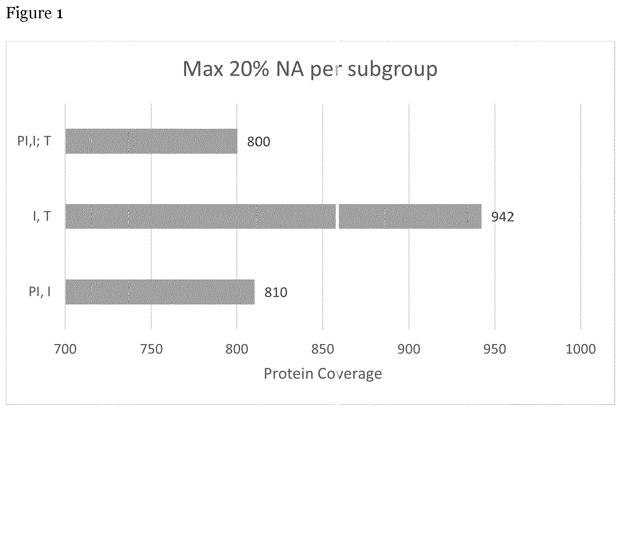

Figure 1 shows a schematic overview over the protein coverage for different comparisons. Number of proteins per comparison with maximum of 20% missing values per subgroup. P = peri-implantitis, I = Healthy implant, T = Healthy tooth.
Figure 2 shows a Partial Least Square Regression Analysis (PLS-DA) of all samples demonstrated a separation of peri-implantitis samples (PI) from healthy samples (implants (I) or teeth (T)). I and T demonstrated no significantly expressed proteins, indicating strong similarities between the crevicular fluid of healthy teeth and implants on the proteome level.

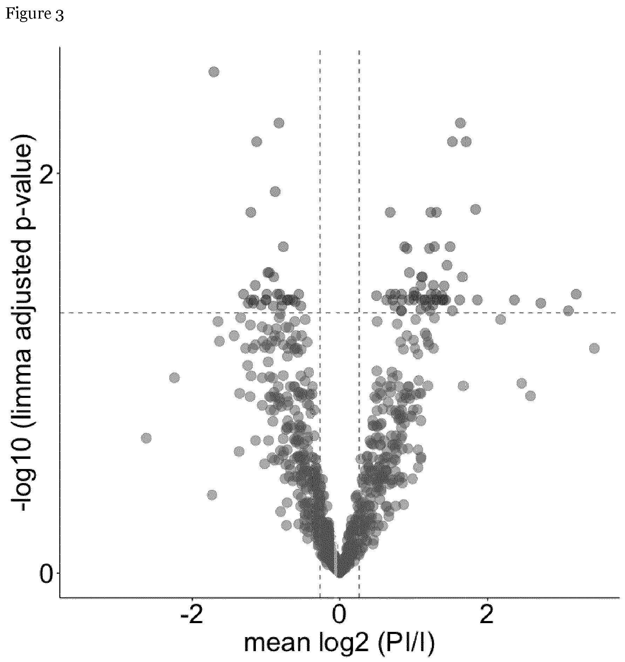

Figure 3 shows a Volcano plot based on *LIMMA* results of peri-implantitis vs healthy implants. log2 cut off: 50%. Adjusted p-value <=0.05. Colored dots represent up- or down-regulated proteins. *LIMMA* demonstrates strong differences between the crevicular fluid protein composition of diseased and healthy implants on the proteome level.

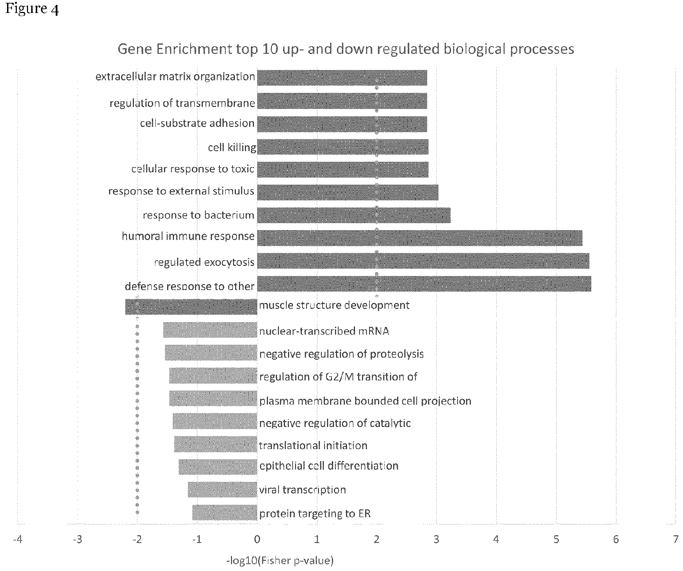

Figure 4 shows the gene ontology-analysis of differentially expressed proteins of peri-implantitis (PI) vs. healthy implants (I). The top ten up- and downregulated biological processes are listed. Upregulated processes: right, downregulated pathways: left. Differentially expressed proteins found in LIMMA (p_{mod}≤0.05) were used for TopGO-Analysis. Meta-Analysis performed using Fisher's method. Results interpreted as statistically signficant if -log10 (Fisher p-value) > 2.0 or < -2 (dashed lines). TopGo-Analysis demonstrated increased immune response pathways in diseased samples.
Figure 5 shows the proportional intensities of semi-specific peptides in different conditions. Kruskal-Wallis and subsequent Dunns post-hoc tests revealed significant differences between peri-implanitits (PI) and healthy teeth (T) (p = 0.0057).
Figure 6 shows a heatmap of distribution of amino acids at different positions in relation to semi-specific cleavage points. *LIMMA* was performed to compare frequency of amino acids no natural abundance. P_{adj} ≤ 0.05. P1 shows increased occurrence of aromatic and aliphatic amino acids.
Figure 7 shows the results on the missingness of bacterial proteins. Especially high missingness in healthy subgroups. Kruskal-Wallis and Dunns post-hoc indicate lower missingness in the PI subgroup. Peri-implanitits (PI), healthy teeth (T) and healthy implants (I).

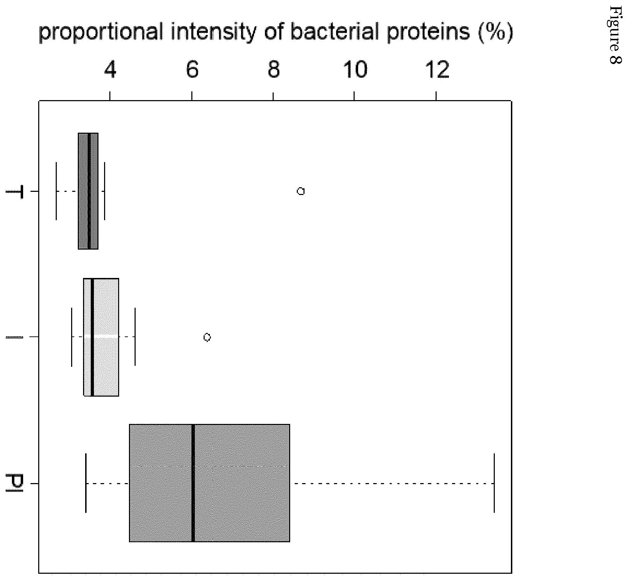

Figure 8 shows the proportional intensities of bacterial proteins in relation to all proteins quantified (bacterial and human). Kruskal-Wallis rank test and Dunns post-hoc test indicate significant differences between PI and healthy subgroups. Peri-implanitits (PI), healthy teeth (T) and healthy implants (I).

### EXAMPLES

In a retrospective cohort study, sulcus fluid samples were analyzed using quantitative proteomics (liquid chromatography-tandem spectrometry, LC-MS/MS). Advantageously, a depletion of highly abundant proteins was not necessary. Using linear models of microarray analysis (LIMMA), the following proteins were found which were significantly (p_{adjusted} < 0.05; mean quantitative difference > 50%) enriched or depleted in the sulcus fluid of the peri-implantitis (compared to healthy implants):
The significantly enriched or depleted proteins are listed in the following two tables. Sulcus also contains cellular debris or cellular components. Classical secreted or shedded proteins from the cell surface are marked. It is interesting to note that in peri-implantitis significantly more classically secreted or shed proteins are enriched than depleted.

**Table 1: Enriched sulcus-proteins in peri-implantitis as identified in the context of the present invention**

| **Uniprot ID** | **Protein** | **Secreted or shedded** |
|---|---|---|
| A0A0C4DH43 | Immunoglobulin heavy variable 2-70D (IGHV70D) | |
| O15511 | Actin-related protein 2/3 complex subunit 5 (Arp2/3 complex 16 kDa subunit) (ARPC5) | |
| O75695 | Protein XRP2 (XRP2) | |
| O75923 | Dysferlin (Dystrophy-associated fer-1-like protein) (DYSF) | |
| O75955 | Flotillin-1 (FLOT1) | x |
| O95445 | Apolipoprotein M (Apo-M) (APOM) | x |
| P00492 | Hypoxanthine-guanine phosphoribosyltransferase (HGPRT) | |
| P00918 | Carbonic anhydrase 2 (Carbonate dehydratase II) (CA2) | |
| P02042 | Hemoglobin subunit delta (Delta-globin) (HBD) | |
| P02750 | Leucine-rich alpha-2-glycoprotein (LRG) | |
| P02753 | Retinol-binding protein 4 (PRBP) | |
| P04040 | Catalase (CAT) | |
| P05107 | Integrin beta-2 (CD antigen CD18) (CD18) | x |
| P05155 | Plasma protease C1 inhibitor (Serpin G1) (SERPING1) | |
| P05164 | Myeloperoxidase (MPO) | x |
| P05546 | Heparin cofactor 2 (HC-II) | |
| P06737 | Glycogen phosphorylase, liver form (PYGL) | |
| P06744 | Glucose-6-phosphate isomerase (GPI) | |
| P07738 | Bisphosphoglycerate mutase (BPGM) | |
| P08133 | Annexin A6 (67 kDa calelectrin) (ANX6) | |
| P10153 | Non-secretory ribonuclease (Eosinophil-derived neurotoxin) (RNASE2) | |
| P11215 | Integrin alpha-M (CD11 antigen-like family member B) (ITGAM) | x |
| P11413 | Glucose-6-phosphate 1-dehydrogenase (G6PD) | |
| P12429 | Annexin A3 (35-alpha calcimedin) (ANX3) | |
| P13716 | Delta-aminolevulinic acid dehydratase (ALADH) | |
| P16083 | Ribosyldihydronicotinamide dehydrogenase (NQO2) | |
| P17213 | Bactericidal permeability-increasing protein (BPI) | x |
| P18084 | Integrin beta-5 (ITGB5) | x |
| P20160 | Azurocidin (Cationic antimicrobial protein CAP37) (AZU1) | |
| Q92820 | Gamma-glutamyl hydrolase (GGH) | |
| P09960 | Leukotriene A-4 hydrolase (LTA-4 hydrolase) (LTA4H) | |
| P25815 | Protein S100-P (Migration-inducing gene 9 protein) (MIG9) | |
| P26583 | High mobility group protein B2 (HMG-2) | |
| P27105 | Stomatin (Erythrocyte band 7 integral membrane protein) (STOM) | |
| P28676 | Grancalcin (GCA) | |
| P29401 | Transketolase (TKT) | |
| P30046 | D-dopachrome decarboxylase (DDT) | |
| P30048 | Thioredoxin-dependent peroxide reductase, mitochondrial (PRDX3) | |
| P30626 | Sorcin (CP-22) (SRI) | |
| P31146 | Coronin-1A (Coronin-like protein A) (CORO1A) | |
| P33241 | Lymphocyte-specific protein 1 (LSP1) | |
| P36222 | Chitinase-3-like protein 1 (39 kDa synovial protein) (CHI3L1) | |
| P37837 | Transaldolase (TALDO1) | |
| P41218 | Myeloid cell nuclear differentiation antigen (MNDA) | x |
| P49913 | Cathelicidin antimicrobial peptide (18 kDa cationic antimicrobial protein) (CAMP) | |
| P52566 | Rho GDP-dissociation inhibitor 2 (Rho GDI 2) (ARHGDIB) | |
| P52790 | Hexokinase-3 (Hexokinase type III) (HK3) | |
| P69905 | Hemoglobin subunit alpha (Alpha-globin) (HBA1) | |
| P80723 | Brain acid soluble protein 1 (BASP1) | |
| Q10588 | ADP-ribosyl cyclase/cyclic ADP-ribose hydrolase 2 (BST1) | |
| Q13228 | Methanethiol oxidase (MTO) | |
| Q14254 | Flotillin-2 (Epidermal surface antigen) (FLOT2) | x |
| Q6P4A8 | Phospholipase B-like 1 (PLBD1) | x |
| Q99439 | Calponin-2 (Calponin H2, smooth muscle) (CNN2) | |
| P67812 | Signal peptidase complex catalytic subunit SEC11A (SEC11A) | |
| Q9H4G4 | Golgi-associated plant pathogenesis-related protein 1 (GAPR-1) | |
| Q9HD89 | Resistin (Adipose tissue-specific secretory factor) (ADSF) | x |
| Q9HDC9 | Adipocyte plasma membrane-associated protein (Protein BSCv) (APMAP) | x |
| Q9Y2J8 | Protein-arginine deiminase type-2 (PAD-H19) (PADI2) | |

**Table 2: Depleted sulcus-proteins in peri-implantitis as identified in the context of the present invention**

| Uniprot ID | Protein | shedded or secreted |
|---|---|---|
| O14579 | Coatomer subunit epsilon (Epsilon-coat protein) (COPE) | |
| O43776 | Asparagine--tRNA ligase, cytoplasmic (Asparaginyl-tRNA synthetase 1) (NARS1) | |
| O75964 | ATP synthase subunit g, mitochondrial (ATP synthase membrane subunit g) (ATP5MG) | |
| O95834 | Echinoderm microtubule-associated protein-like 2 (EMAP-2) | |
| P04439 | HLA class I histocompatibility antigen, A alpha chain (Human leukocyte antigen A) (HLA-A) | |
| P07339 | Cathepsin D (CTSD) | X |
| P07355 | Annexin A2 (ANX2) | |
| P04632 | Calpain small subunit 1(Calcium-activated neutral proteinase small subunit) (CAPNS1) | |
| P07858 | Cathepsin B (APP secretase) (CTSB) | |
| P07900 | Heat shock protein HSP 90-alpha (HSP90AA1) | |
| P09758 | Tumor-associated calcium signal transducer 2 (Cell surface glycoprotein Trop-2) (TACSTD2) | |
| P10599 | Thioredoxin (Trx) (TXN) | |
| P13073 | Cytochrome c oxidase subunit 4 isoform 1, mitochondrial (Cytochrome c oxidase polypeptide IV) (COX4I1) | |
| P13797 | Plastin-3 (T-plastin) (PLS3) | |
| P15311 | Ezrin (Cytovillin) (EZR) | |
| P16152 | Carbonyl reductase (CBR1) | |
| P18124 | 60S ribosomal protein L7 (RPL7) | |
| P30086 | Phosphatidylethanolamine-binding protein 1 (PEBP-1) | |
| P31949 | Protein S100-A11 (Calgizzarin) (S100A11) | |
| P40925 | Malate dehydrogenase, cytoplasmic (Cytosolic malate dehydrogenase) (MDH1) | |
| P49189 | 4-trimethylaminobutyraldehyde dehydrogenase (TMABA-DH) (ALDH9A1) | |
| P54920 | Alpha-soluble NSF attachment protein (SNAP-alpha) (NAPA) | |
| Q00325 | Phosphate carrier protein, mitochondrial (SLC25A3) | |
| Q13200 | 26S proteasome non-ATPase regulatory subunit 2 (PSMD2) | |
| Q15149 | Plectin (PCN) | |
| Q3LXA3 | Triokinase/FMN cyclase (TKFC) | |
| Q99733 | Nucleosome assembly protein 1-like 4 (NAP-2) | |
| Q99798 | Aconitate hydratase, mitochondrial (Aconitase) (ACO2) | |
| Q9UHY7 | Enolase-phosphatase E1 (ENOPH1) | |
| Q9UK76 | Jupiter microtubule associated homolog 1 (Androgen-regulated protein 2) (JPT1) | |
| Q9UL46 | Proteasome activator complex subunit 2 (11S regulator complex subunit beta) (PSME2) | |

The study that provided the basis for the present invention was approved by the ethics committee of the University Medical Center Freiburg, Germany (Ethik-Kommission Albert-Ludwigs-Universität, Freiburg, Votum 337/04). Before enrollment, the patients received information regarding the purpose of the study and signed an informed consent. All patients were consecutively enrolled between 2020 and 2021 (Department of Oral- and Craniomaxillofacial Surgery / Translational Implantology, University Medical Center Freiburg). The study was performed in accordance with the Helsinki Declaration of 1964, as revised in 2013. The study was conducted in accordance with the SRQR (Standards for Reporting Qualitative Research) guidelines.

### Study cohort

Patients with at least one diseased implant were included. Peri-implantitis is defined according to the current international guideline with peri-implant pocket depth ≥ 6 mm, ≥ 3 mm peri-implant radiological bone loss with bleeding and / or suppuration on probing (Berglundh 2018). Minimum age was 18 years. Prosthetically restored Implants were included which had received the restoration > 12 months prior. Screw retained and cemented restorations were included.

Patients were excluded from the study if one of the following conditions was met: diabetes, immunosuppression or immunosuppressive medication, current radiation of the head or neck area, or oral mucosal diseases (e.g. erosive lichen planus), bisphosphonate or antiresorptive agent intake, current untreated periodontal disease or gingivitis, pocket depth of ≥ 4 mm at the teeth immediately next to the dental implant to be examined, severe bruxism and patients with inadequate oral hygiene or not motivated to provide adequate oral care at home, current intake of antibiotics or local application of antibiotics. Also, patients were only included if no peri-implantitis treatment had been performed before.

### PICF sample collection

PICF sample collection was performed as described (Iglhaut et al. 2021). Briefly, after gentle air-drying, peri-implant sulcus fluid samples were obtained at the mesio-buccal aspect of the target implant site, as well as at the healthy tooth and healthy implant, with sterile paper strips (Periopaper, Oraflow Inc., Hewlett, NY, USA). The strips were placed into the sulcus with a minimum depth of 1-2 mm for 30 sec. The procedure was performed three times with fresh strips. Strips were instantly collected in cryotubes and frozen using liquid nitrogen. Subsequently, samples were transported on ice and stored at - 80°C.

### Sample Preparation for LC-MS/MS

Samples were thawed and eluted in 150 µl of 0.1 % acid labile surfactant in 0.1 M HEPES. After sonification for 15 min, samples were incubated at 90 °C for 30 min. Reduction was performed by adding a final concentration of 5 mM DTT and incubating for 30 min at 37 °C, followed by alkylation in 15 mM chloracetamide and again incubating for 30 min at 37 °C in the dark (Krauspe et al. 2021). For tryptic digestion 1 µg LysC (Lysyl Endopeptidase^{®}, Mass spectrometry grade, Fujifilm/WAKO) was added and incubated for 2 hours at 37 °C. Further, 1 µg of sequencing grade trypsin (Mass spectrometry grade, Promega) was added. Digestion was performed over night at 37 °C. By adding TFA to a final concentration of 2 % the acid labile surfactant was precipitated and after centrifugation for 15 minutes at 15000 rpm (21130 rcf) the supernatant was transferred to iST-columns (Preomics). Desalting was performed following manufacturer's instructions. In short, columns were washed four times with two kinds of washing buffers (washing-buffer I: 1 % TFA in 2-propanol; washing-buffer II: 0.2 % TFA in ddH₂O). Peptides were eluted into low bind Eppendorf-tubes with 200 µl of 2 % triethylamine in 80 % acetonitrile (in water). Peptide amount was measured with a bicinchoninic acid assay. To generate a spectral library, 5 µg of each sample with more than 10 µg of peptides were pooled. Until MS, samples were dried and stored at - 80 °C.

### MS Measurements

All samples were analyzed on an Orbitrap Q-Exactive Plus mass spectrometer (Thermo Scientific, San Jose, CA, US) coupled to an Easy nanoLCTM 100 UHPLC (Thermo Scientific, San Jose, CA, US). 0.5 µg of peptides were injected and iRT peptides (Biognosys, Schlieren, Switzerland) were added before MS measurements. Analytical columns were self-packed with C18-coated silica beads (Reprosil Pur C18-AQ, d = 3 Â) (Dr. Maisch HPLC GmbH, Ammerbusch, Germany). Buffer A was 0.1 % formic acid, buffer B was 0.1 % formic acid in 80 % Acetonitrile.

To generate an extensive spectral library, the pooled sample was injected for MS six consecutive times with different mass ranges (mass ranges: 400-500, 500-600, 600-700, 700-800, 800-900, 900-1000 Th). A two-step gradient (110 min) was applied with Buffer B increasing from 8 % to 43 % over 90 min and from 43 to 65 % in 20 min. The mass spectrometer was set to DIA-mode with the following settings: AGC target: 1e6; maximum injection time: 80ms; NCE: 25 and 30; MS1 resolution: 70,000; MS2 resolution: 17,500.

Samples were measured on the same column in DIA-mode. Mass-to-charge range was 400 - 1000 m/z, two cycles of 24 m/z widows were applied with a 50 % shift in between cycles.

### Generation of Spectral Library and Sample Analysis

Processing of RAW-files from MS-measurements was performed using DIA-NN (v. 1.7.12) (Demichev et al. 2020). An UNIPROT human database was used, (downloaded in June 2021) in combination with iRT protein sequences (Biogynesis) to create a spectral library. In DIA-NN, tryptic digestion was chosen, allowing for one missed cleavage site. Minimum peptide length was set to seven. Analysis allowed for cysteine carbamidomethylation. Other modifications were not included. Precursor charge range was set to 1 - 4 and precursor m/z range was 300-1800 Th. Fragment ion m/z range was set to 200 - 1800 Th. Precursor false discovery rate was 0.01. The spectral library was subsequently used for sample analysis.

Same settings regarding FDR, peptide length and peptide modification were used for analysis of individual samples. In addition, match between runs was enabled. All samples were analyzed in the same run. The protein groups (PG) normalized output of DIA-NN was used for further analysis

### Microbiome Analysis

For identification of bacterial proteins, a second DIA-NN analysis was performed. Genomes of bacteria found in the oral cavity were downloaded from the NIH Human Microbiome Project Reference Genome Sequence Database in September 2021. The reviewed human database that was used for the detection of human proteins was combined with the microbiome genomes. The fasta-file compiling both human and bacterial proteins was imported into DIA-NN for spectral library generation. Otherwise, the same settings as listed above applied for library generation and subsequent sample analysis. For further analysis, only proteotyptic peptides were allowed.

### Identification of Semi-specific Peptides

In a third DIA-NN analysis, *in silico* library generation with DIA-NN based on a reviewed human fasta-database (downloaded from Uniprot in November 2020) was performed. In short, library free search was enabled with a fragment ion m/z-range between 200-1800 Th. N-terminal methionine excision was enabled, *in silico* digestion with cuts at K and R and no missed cleavages were allowed. For analysis of samples, the same settings as mentioned above applied. For identification of semi-specific and non-tryptic peptides, all peptides found were searched against the fasta-file that had been generated previously in DIA-NN.

### Statistical Analysis

For statistical analysis, RStudio was used (v. 1.4.1103-4) (R Foundation for Statistical Computing). Contaminants, iRTs and reverse entries were removed. For further analysis, only those proteins found in 20 % or more samples were considered. M/Z-values were log2-transformed and median centered. Partial Least Squares Regression Analysis (PLS-DA) and Linear Models for Microarray Data (LIMMA) (Ritchie et al. 2015) were applied, comparing all three groups (teeth, healthy implant, peri-implantitis), as well as subgroups healthy tooth vs. healthy implant and healthy implant vs. peri-implantitis. The adjusted p-value was set to p_{adj} = 0.05 and only proteins with log2-changes of +- 50 % were considered as up- or downregulated.

To evaluate how frequently bacterial proteins were identified per condition, missingness of proteins was calculated. Missingness represents the proportion of samples of one condition in which a given protein was not identified. High missingness in one condition indicates that the protein was identified only in a small number of samples.

### Results

38 sites of 14 patients (6 female, 8 male, age range 52 - 85 years, median age was 70.7) were included in the study. Patients were diagnosed with peri-implantitis according to the above-mentioned criteria. The inventors aimed at including diseased implants and healthy implants of the same patients: six patients with samples of peri-implantitis affected implants (PI), healthy implants (I) and healthy teeth (T), three patients with PI and I, three patients with PI and T and two patients with no other sample than PI were included. At total, 17 PI-, 12 I- and 9 T-samples were included.

Three patients reported a history of smoking, two patients had been treated with radiation in the head and neck area after resection of an adenoid cystic carcinoma or tongue carcinoma. Here, radiation was > 10 years ago.

### Human Proteome Coverage

2332 different human proteins were identified across all samples. Protein identifications per sample ranged from 724 to 1804 with a false discovery rate < 1%. Average proteome coverage per condition revealed significantly lower protein counts for the PI-subgroup when compared to healthy implants (Kruskal-Wallis and subsequent Dunns post-hoc test (p_{adj} = 0.021)). No significant differences in protein coverage were found between PI and I or between healthy conditions (T and I). The inventors identified a coreset of 357 proteins found in all samples. For further analysis the inventors only considered proteins found and quantified in at least 80% of all samples of one subgroup (max. three missing values in subgroup PI, max. two missing values in subgroup I, max. one missing value in subgroup T).

### Partial Least Square Regression Analysis (PLS-DA) and LIMMA Indicate Distinct Crevicular Fluid Proteome Profiles for Peri-implantitis as Compared to Healthy Teeth and Healthy Implants

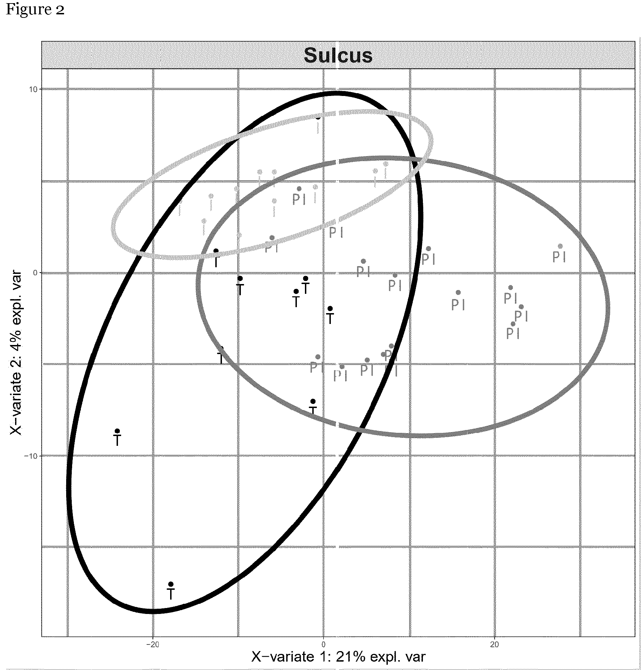

To check for clustering of samples a Partial Least Square Regression Analysis (PLS-DA) was performed (Figure 2). PLS-DA resembles a supervised method to distinguish between expected and observed variables. Here, healthy implants were identified as a subgroup of healthy teeth on the proteome level. Peri-implantitis is separated from healthy teeth and healthy implants. This finding underlines a distinguishable proteome profile for crevicular fluid stemming from peri-implantitis as compared to healthy implants or healthy teeth.

For further statistical analysis, *LIMMA* was applied to the following comparisons: PI vs. I; T vs. I. The latter revealed no significantly expressed proteins, indicating strong similarities between the crevicular fluid of healthy teeth and implants on the proteome level. When comparing PI to healthy implants, 78 proteins were found either upregulated or downregulated in PI with statistical significance of adjusted p-value ≤ 0.05. Here, a variety of inflammatory proteins (dysferlin, leukotriene A-4 hydrolase (LTA4H), S100P and others) and proteins related to bacterial defense (myeloperoxidase, bactericidal permeability-increasing protein, azurocidin and others) were identified.

### Matrix Metalloproteinases-8 and -9

In total, 21 proteases related to inflammation were identified and quantified across all samples. Both MMP8 and MMP9 were found in all samples with elevated levels in the PI subgroup. Moderated p-value (PI vs. I) for MMP8 was p = 0.037 and p = 0.042 for MMP9 (Table 2). Although their adjusted p-values exceeded p_{adj} = 0.05 (p_{adj} < 0.2; PI *vs.* I), MMP8 and -9 remain of interest in the context of peri-implantitis since they are endoproteolytic enzymes with collagenolytic / gelatinolytic activity. Leukotriene A-4 hydrolase (LTA4H) and gamma-glutamyl hydrolase were found upregulated in PI with statistical significance (p_{adj} ≤ 0.05), yet these enzymes are not considered prototypical endoproteases.

### Gene Ontology Enrichment Analysis

A gene ontology (TopGO) enrichment was performed to categorize proteins identified as differentially expressed. Proteins up- or downregulated around diseased implants when compared to healthy implants were included (log2-fold change of +- 50 %, p_{mod} ≤ 0.05). TopGo enrichment revealed several immunological pathways to be upregulated with strongest manifestation in "humoral immune response", "defense response to other organism" and "regulated exocytosis".

### Endogenous Proteolysis

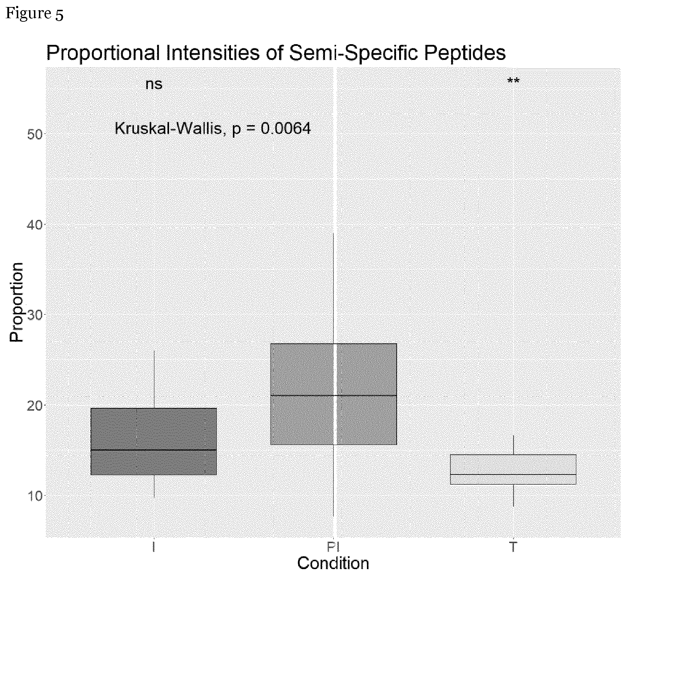

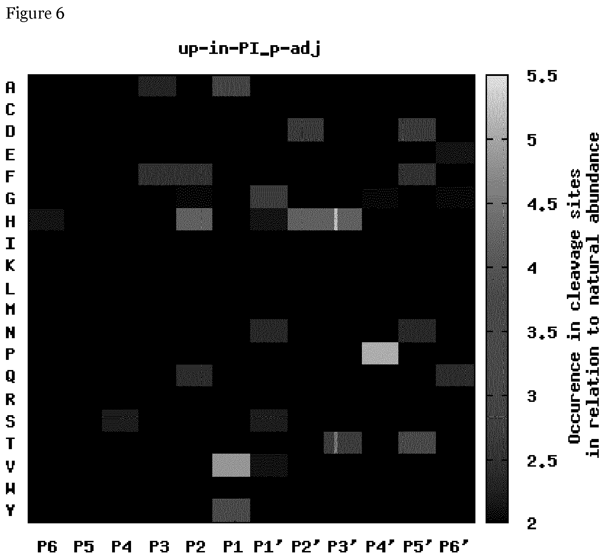

In classical bottom-up proteomics using trypsination, endogenous proteolysis manifests in the form of truncated, so-called semi-specific peptides (Fahrner et al. 2021). To study endogenous proteolysis in peri-implantitis, the inventors performed an additional data analysis step with a spectral library that also represents N- or C-terminally truncated peptides of the human proteome. The inventors note that this approach cannot capture endogenous proteolysis with trypsin-like specificity. 10566 different peptides were identified, 2362 of which were of semi-specific nature. Proportional intensities of semitryptic peptides were calculated per sample (Figure 5). Increased proportional intensities of semi-specific peptides were found in PI with statistical significance compared to healthy teeth (p = 0.0057), indicating that peri-implantitis lesions possess higher endogenous proteolytic activity. To evaluate possible underlying proteases, six amino acids before and after the semi-specific cleavage point were evaluated in relation to their natural abundance (Figure 6). In position P1 of the peptide, aliphatic (Valin) and aromatic (Tyrosine) amino acids occurred more frequently (up to 5 times more often). Up- and downstream of the cleavage point aliphatic amino acids overweight (Phenylaniline and Histidine). Therefore, the cleavage pattern observed is not dominated by activity of MMPs, but rather by proinflammatory proteases such as cathepsin G or neutrophil elastase which prefer aliphatic amino acids in the P1 position.

### Bacterial Proteins

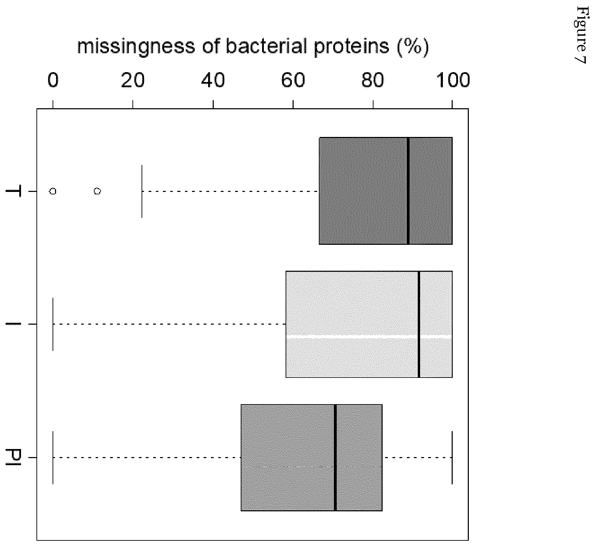

Further, the inventors investigated the presence of bacterial proteins in the crevicular fluid. A spectral library representing the human proteome supplemented with the proteome of the human oral cavity as defined by the NIH Human Microbiome Project Reference Genome Sequence Database was created. This approach only serves as a proxy for the human oral microbiome, this approach does not capture species that are absent from these data sources. 334 bacterial proteins were identified and quantified. To evaluate differences in intensities of bacterial proteins, proportional intensities of all bacterial proteins found were calculated per sample (Figure 7). Kruskal-Wallis rank test revealed significant differences between conditions (PI, I and T) and Dunns post-hoc-test indicated significant differences between PI and both healthy conditions (T and I). Furthermore, missingness of bacterial proteins was calculated. In the healthy conditions, missingness was especially high with nearly no proteins identified in all samples. Missingness was lower in the peri-implantitis subgroup, yet still more than 75% of all proteins were missing in more than half of all samples. Again, Kruskal-Wallis rank test and subsequent Dunns post-hoc-test showed significant differences between the diseased and both healthy conditions (Figure 7).

### Literature as cited

Ahadi, Sara; Zhou, Wenyu; Schüssler-Fiorenza Rose, Sophia Miryam; Sailani, M. Reza; Contrepois, Kévin; Avina, Monika et al. (2020): Personal aging markers and ageotypes revealed by deep longitudinal profiling. In: Nature medicine 26 (1), S. 83-90. DOI: 10.1038/s41591-019-0719-5.
Alassiri, Saeed; Parnanen, Pirjo; Rathnayake, Nilminie; Johannsen, Gunnar; Heikkinen, Anna-Maria; Lazzara, Richard et al. (2018): The Ability of Quantitative, Specific, and Sensitive Point-of-Care/Chair-Side Oral Fluid Immunotests for aMMP-8 to Detect Periodontal and Peri-Implant Diseases. In: Disease markers 2018, S. 1306396. DOI: 10.1155/2018/1306396.
Alassy, Hatem; Parachuru, Praveen; Wolff, Larry (2019): Peri-Implantitis Diagnosis and Prognosis Using Biomarkers in Peri-Implant Crevicular Fluid: A Narrative Review. In: Diagnostics (Basel, Switzerland) 9 (4). DOI: 10.3390/diagnostics9040214.
Albrektsson, Tomas; Canullo, Luigi; Cochran, David; Bruyn, Hugo de (2016): "Peri-Implantitis": A Complication of a Foreign Body or a Man-Made "Disease". Facts and Fiction. In: Clinical implant dentistry and related research 18 (4), S. 840-849. DOI: 10.1111/cid.12427.
Andrucioli, Marcela Cristina Damião; Matsumoto, Mirian Aiko Nakane; Fukada, Sandra Yasuyo; Saraiva, Maria Conceição Pereira; Bergamo, Ana Zilda Nazar; Romano, Fábio Lourengo et al. (2019): Quantification of pro-inflammatory cytokines and osteoclastogenesis markers in successful and failed orthodontic mini-implants. In: Journal of applied oral science: revista FOB 27, e20180476. DOI: 10.1590/1678-7757-2018-0476.
Arakawa, Hikaru; Uehara, Junji; Hara, Emilio Satoshi; Sonoyama, Wataru; Kimura, Aya; Kanyama, Manabu et al. (2012): Matrix metalloproteinase-8 is the major potential collagenase in active peri-implantitis. In: Journal of prosthodontic research 56 (4), S. 249-255. DOI: 10.1016/j.jpor.2012.07.002.
Arias-Bujanda, Nora; Regueira-Iglesias, Alba; Balsa-Castro, Carlos; Nibali, Luigi; Donos, Nikos; Tomás, Inmaculada (2019): Accuracy of single molecular biomarkers in gingival crevicular fluid for the diagnosis of periodontitis: A systematic review and meta-analysis. In: Journal of clinical periodontology 46 (12), S. 1166-1182. DOI: 10.1111/jcpe.13188.
Arumugam, Thiruvengadam; Simeone, Diane M.; Schmidt, Ann Marie; Logsdon, Craig D. (2004): S100P stimulates cell proliferation and survival via receptor for activated glycation end products (RAGE). In: The Journal of biological chemistry 279 (7), S. 5059-5065. DOI: 10.1074/jbc.M310124200.
Baliban, Richard C.; Sakellari, Dimitra; Li, Zukui; DiMaggio, Peter A.; Garcia, Benjamin A.; Floudas, Christodoulos A. (2012): Novel protein identification methods for biomarker discovery via a proteomic analysis of periodontally healthy and diseased gingival crevicular fluid samples. In: Journal of clinical periodontology, S. 203-212. DOI: 10.1111/j.1600-051X.2011.01805.x.
Bao, Kai; Li, Xiaofei; Kajikawa, Tetsuhiro; Toshiharu, Abe; Selevsek, Nathalie; Grossmann, Jonas et al. (2020): Pressure Cycling Technology Assisted Mass Spectrometric Quantification of Gingival Tissue Reveals Proteome Dynamics during the Initiation and Progression of Inflammatory Periodontal Disease. In: Proteomics 20 (3-4), e1900253. DOI: 10.1002/pmic.201900253.
Basegmez, Cansu; Yalcin, Serdar; Yalcin, Funda; Ersanli, Selim; Mijiritsky, Eitan (2012): Evaluation of periimplant crevicular fluid prostaglandin E2 and matrix metalloproteinase-8 levels from health to periimplant disease status: a prospective study. In: Implant dentistry 21 (4), S. 306-310. DOI: 10.1097/ID.0b013e3182588408.
Berglundh, Tord; Armitage, Gary; Araujo, Mauricio G.; Avila-Ortiz, Gustavo; Blanco, Juan; Camargo, Paulo M. et al. (2018): Peri-implant diseases and conditions: Consensus report of workgroup 4 of the 2017 World Workshop on the Classification of Periodontal and Peri-Implant Diseases and Conditions. In: Journal of periodontology 89 Suppl 1, S313-S318. DOI: 10.1002/JPER.17-0739.
Bostanci, N.; Bao, K.; Wahlander, A.; Grossmann, J.; Thurnheer, T.; Belibasakis, G. N. (2015): Secretome of gingival epithelium in response to subgingival biofilms. In: Molecular oral microbiology, S. 323-335. DOI: 10.1111/omi.12096.
Bostanci, N.; Heywood, W.; Mills, K.; Parkar, M.; Nibali, L.; Donos, N. (2010): Application of label-free absolute quantitative proteomics in human gingival crevicular fluid by LC/MS E (gingival exudatome). In: Journal of proteome research 9 (5), S. 2191-2199. DOI: 10.1021/pr900941z.
Bruderer, Roland; Bernhardt, Oliver M.; Gandhi, Tejas; Miladinović, Saša M.; Cheng, Lin-Yang; Messner, Simon et al. (2015): Extending the limits of quantitative proteome profiling with data-independent acquisition and application to acetaminophen-treated three-dimensional liver microtissues. In: Molecular & cellular proteomics : MCP 14 (5), S. 1400-1410. DOI: 10.1074/mcp.M114.044305.
Campanelli, D.; Detmers, P. A.; Nathan, C. F.; Gabay, J. E. (1990): Azurocidin and a homologous serine protease from neutrophils. Differential antimicrobial and proteolytic properties. In: The Journal of clinical investigation 85 (3), S. 904-915. DOI: 10.1172/JCI114518.
Canullo, Luigi; Schlee, Markus; Wagner, Wilfried; Covani, Ugo (2015): International Brainstorming Meeting on Etiologic and Risk Factors of Peri-implantitis, Montegrotto (Padua, Italy), August 2014. In: The International journal of oral & maxillofacial implants 30 (5), S. 1093-1104. DOI: 10.11607/jomi.4386.
Carcuac, O.; Berglundh, T. (2014): Composition of human peri-implantitis and periodontitis lesions. In: Journal of dental research 93 (11), S. 1083-1088. DOI: 10.1177/0022034514551754.
Carcuac, Olivier; Derks, Jan; Abrahamsson, Ingemar; Wennström, Jan L.; Berglundh, Tord (2020): Risk for recurrence of disease following surgical therapy of peri-implantitis-A prospective longitudinal study. In: Clinical oral implants research 31 (11), S. 1072-1077. DOI: 10.1111/clr. 13653.
Demichev, Vadim; Messner, Christoph B.; Vernardis, Spyros I.; Lilley, Kathryn S.; Ralser, Markus (2020): DIA-NN: neural networks and interference correction enable deep proteome coverage in high throughput. In: Nature methods 17 (1), S. 41-44. DOI: 10.1038/s41592-019-0638-x.
Derks, Jan; Schaller, Dennis; Håkansson, Jan; Wennström, Jan L.; Tomasi, Cristiano; Berglundh, Tord (2016): Peri-implantitis - onset and pattern of progression. In: Journal of clinical periodontology 43 (4), S. 383-388. DOI: 10.1111/jcpe. 12535.
Duarte, P. M.; Serrão, C. R.; Miranda, T. S.; Zanatta, L. C. S.; Bastos, M. F.; Faveri, M. et al. (2016): Could cytokine levels in the peri-implant crevicular fluid be used to distinguish between healthy implants and implants with peri-implantitis? A systematic review. In: Journal of periodontal research 51 (6), S. 689-698. DOI: 10.1111/jre.12354. Durrani, Farhan; Singh, Royana (2015): Myeloperoxidase level around dental implants as an indicator of an inflammatory process. In: Indian journal of dentistry 6 (1), S. 2-6. DOI: 10.4103/0975-962X.151688.
Esberg, Anders; Isehed, Catrine; Holmlund, Anders; Lundberg, Pernilla (2019): Peri-implant crevicular fluid proteome before and after adjunctive enamel matrix derivative treatment of peri-implantitis. In: Journal of clinical periodontology 46 (6), S. 669-677. DOI: 10.1111/jcpe.13108.
Esposito, Marco; Grusovin, Maria Gabriella; Worthington, Helen V. (2012): Treatment of peri-implantitis: what interventions are effective? A Cochrane systematic review. In: European journal of oral implantology 5 Suppl, S21-41.
Fahrner, Matthias; Kook, Lucas; Fröhlich, Klemens; Biniossek, Martin L.; Schilling, Oliver (2021): A Systematic Evaluation of Semispecific Peptide Search Parameter Enables Identification of Previously Undescribed N-Terminal Peptides and Conserved Proteolytic Processing in Cancer Cell Lines. In: Proteomes 9 (2). DOI: 10.3390/proteomes9020026.
Fretwurst, T.; Nelson, K.; Tarnow, D. P.; Wang, H-L; Giannobile, W. V. (2018): Is Metal Particle Release Associated with Peri-implant Bone Destruction? An Emerging Concept. In: Journal of dental research 97 (3), S. 259-265. DOI: 10.1177/0022034517740560.
Fretwurst, Tobias; Buzanich, Guenter; Nahles, Susanne; Woelber, Johan Peter; Riesemeier, Heinrich; Nelson, Katja (2016): Metal elements in tissue with dental peri-implantitis: a pilot study. In: Clinical oral implants research 27 (9), S. 1178-1186. DOI: 10.1111/clr.12718.
Fretwurst, Tobias; Garaicoa-Pazmino, Carlos; Nelson, Katja; Giannobile, William V.; Squarize, Cristiane H.; Larsson, Lena; Castilho, Rogerio M. (2020): Characterization of macrophages infiltrating peri-implantitis lesions. In: Clinical oral implants research 31 (3), S. 274-281. DOI: 10.1111/clr.13568.
Fretwurst, Tobias; Müller, Janina; Larsson, Lena; Bronsert, Peter; Hazard, Derek; Castilho, Rogerio M. et al. (2021): Immunohistological composition of peri-implantitis affected tissue around ceramic implants - A pilot study. In: Journal of periodontology. DOI: 10. 1 002/JPER.20-0 169.
Furtmüller, P. G.; Burner, U.; Obinger, C. (1998): Reaction of myeloperoxidase compound I with chloride, bromide, iodide, and thiocyanate. In: Biochemistry 37 (51), S. 17923-17930. DOI: 10.1021/bi9818772.
Gianazza, Elisabetta; Miller, Ingrid; Palazzolo, Luca; Parravicini, Chiara; Eberini, Ivano (2016): With or without you - Proteomics with or without major plasma/serum proteins. In: Journal of proteomics 140, S. 62-80. DOI: 10.1016/j.jprot.2016.04.002.
Gillet, Ludovic C.; Navarro, Pedro; Tate, Stephen; Röst, Hannes; Selevsek, Nathalie; Reiter, Lukas et al. (2012): Targeted data extraction of the MS/MS spectra generated by data-independent acquisition: a new concept for consistent and accurate proteome analysis. In: Molecular & cellular proteomics : MCP 11 (6), 0111.016717. DOI: 10.1074/mcp.O111.016717.
Gürlek, Önder; Gümü 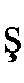 , Pinar; Nile, Christopher J.; Lappin, David F.; Buduneli, Nurcan (2017): Biomarkers and Bacteria Around Implants and Natural Teeth in the Same Individuals. In: Journal of periodontology 88 (8), S. 752-761. DOI: 10.1902/jop.2017.160751.
Iglhaut, Gerhard; Salomon, Sebastian; Fretwurst, Tobias; Thomas, Peter; Endres, Janina; Kessler, Selina; Summer, Burkhard (2021): Cross-sectional evaluation of clinical and immunological parameters at partially microgrooved vs machined abutments in humans. In: International journal of implant dentistry 7 (1), S. 46. DOI: 10.1186/s40729-021-00329-8.
Jiang, Hongfei; Hu, Hang; Tong, Xiaomei; Jiang, Qiuhong; Zhu, Haiyan; Zhang, Songying (2012): Calcium-binding protein S100P and cancer: mechanisms and clinical relevance. In: Journal of cancer research and clinical oncology 138 (1), S. 1-9. DOI: 10.1007/s00432-011-1062-5.
Jiang, Lihua; Wang, Meng; Lin, Shin; Jian, Ruiqi; Li, Xiao; Chan, Joanne et al. (2020): A Quantitative Proteome Map of the Human Body. In: Cell 183 (1), 269-283.e19. DOI: 10.1016/j.cell.2020.08.036.
Karatas, Ozkan; Balci Yuce, Hatice; Taskan, Mehmet Murat; Gevrek, Fikret; Lafci, Emre; Kasap, Hayrunnisa (2020): Histological evaluation of peri-implant mucosal and gingival tissues in peri-implantitis, peri-implant mucositis and periodontitis patients: a cross-sectional clinical study. In: Acta Odontologica Scandinavica 78 (4), S. 241-249. DOI: 10.1080/00016357.2019.1691256.
Kelstrup, Christian D.; Bekker-Jensen, Dorte B.; Arrey, Tabiwang N.; Hogrebe, Alexander; Harder, Alexander; Olsen, Jesper V. (2018): Performance Evaluation of the Q Exactive HF-X for Shotgun Proteomics. In: Journal of proteome research 17 (1), S. 727-738. DOI: 10.1021/acs.jproteome.7b00602.
Kensara, Anmar; Hefni, Eman; Williams, Mary Ann; Saito, Hanae; Mongodin, Emmanuel; Masri, Radi (2021): Microbiological Profile and Human Immune Response Associated with Peri-Implantitis: A Systematic Review. In: Journal of prosthodontics : official journal of the American College of Prosthodontists 30 (3), S. 210-234. DOI: 10.1111/jopr.13270.
Khosla, S. (2001): Minireview: the OPG/RANKL/RANK system. In: Endocrinology 142 (12), S. 5050-5055. DOI: 10.1210/endo.142.12.8536.
Korkmaz, Brice; Horwitz, Marshall S.; Jenne, Dieter E.; Gauthier, Francis (2010): Neutrophil elastase, proteinase 3, and cathepsin G as therapeutic targets in human diseases. In: Pharmacological reviews 62 (4), S. 726-759. DOI: 10.1124/pr.110.002733. Krakauer, Teresa (2008): Nuclear factor-kappaB: fine-tuning a central integrator of diverse biologic stimuli. In: International reviews of immunology 27 (5), S. 286-292. DOI: 10.1080/08830180802317957.
Krauspe, Vanessa; Fahrner, Matthias; Spät, Philipp; Steglich, Claudia; Frankenberg-Dinkel, Nicole; Maček, Boris et al. (2021): Discovery of a small protein factor involved in the coordinated degradation of phycobilisomes in cyanobacteria. In: Proceedings of the National Academy of Sciences of the United States of America 118 (5). DOI: 10.1073/pnas.2012277118.
Kwon, Ho Jeong; Fehniger, Thomas E.; Marko-Varga, György (2016): Building the basis for proteomics in personalized medicine for targeted treatment. In: Clinical and translational medicine 5 (1), S. 19. DOI: 10.1186/s40169-016-0096-3.
Lafaurie, Gloria Inés; Sabogal, Maria Alejandra; Castillo, Diana Marcela; Rincón, Maria Victoria; Gómez, Luz Amparo; Lesmes, Yamil Augusto; Chambrone, Leandro (2017): Microbiome and Microbial Biofilm Profiles of Peri-Implantitis: A Systematic Review. In: Journal of periodontology 88 (10), S. 1066-1089. DOI: 10.1902/jop.2017.170123.
Le Tourneau, Christophe; Borcoman, Edith; Kamal, Maud (2019): Molecular profiling in precision medicine oncology. In: Nature medicine 25 (5), S. 711-712. DOI: 10.1038/s41591-019-0442-2.
Loos, Bruno G.; Tjoa, Stanley (2005): Host-derived diagnostic markers for periodontitis: do they exist in gingival crevice fluid? In: Periodontology 2000 39, S. 53-72. DOI: 10.1111/j .1600-0757.2005.00129.x.
Ludwig, Christina; Gillet, Ludovic; Rosenberger, George; Amon, Sabine; Collins, Ben C.; Aebersold, Ruedi (2018): Data-independent acquisition-based SWATH-MS for quantitative proteomics: a tutorial. In: Molecular systems biology 14 (8), e8126. DOI: 10.15252/msb.20178126.
Marinho, Marcelo C.; Pacheco, Ana Beatriz F.; Costa, Giovani C. V.; Ortiz, Nina D.; Zajdenverg, Lenita; Sansone, Carmelo (2019): Quantitative gingival crevicular fluid proteome in type 2 diabetes mellitus and chronic periodontitis. In: Oral diseases 25 (2), S. 588-595. DOI: 10.1111/odi.12996.
Maxeiner, Sebastian; Shi, Nian; Schalla, Carmen; Aydin, Guelcan; Hoss, Mareike; Vogel, Simon et al. (2015): Crucial role for the LSP1-myosin1e bimolecular complex in the regulation of Fcγ receptor-driven phagocytosis. In: Molecular biology of the cell 26 (9), S. 1652-1664. DOI: 10.1091/mbc.E14-05-1005.
Mombelli, Andrea; Décaillet, Fabien (2011): The characteristics of biofilms in peri-implant disease. In: Journal of clinical periodontology 38 Suppl 11, S. 203-213. DOI: 10.1111/j.1600-051X.2010.01666.x.
Nalmpantis, Dimitrios; Gatou, Asimina; Fragkioudakis, Ioannis; Margariti, Apostolia; Skoura, Lemonia; Sakellari, Dimitra (2020): Azurocidin in gingival crevicular fluid as a potential biomarker of chronic periodontitis. In: Journal of periodontal research 55 (2), S. 209-214. DOI: 10.1111/jre.12703.
Nguyen, Trang; Sedghi, Lea; Ganther, Sean; Malone, Erin; Kamarajan, Pachiyappan; Kapila, Yvonne L. (2020): Host-microbe interactions: Profiles in the transcriptome, the proteome, and the metabolome. In: Periodontology 2000 82 (1), S. 115-128. DOI: 10.1111/prd.12316.
Palanissami, Gowri; Paul, Solomon F. D. (2018): RAGE and Its Ligands: Molecular Interplay Between Glycation, Inflammation, and Hallmarks of Cancer-a Review. In: Hormones & cancer 9 (5), S. 295-325. DOI: 10.1007/s12672-018-0342-9.
Persson, G. Rutger; Renvert, Stefan (2014): Cluster of bacteria associated with peri-implantitis. In: Clinical implant dentistry and related research 16 (6), S. 783-793. DOI: 10.1111/cid.12052.
Rakic, Mia; Lekovic, Vojislav; Nikolic-Jakoba, Natasa; Vojvodic, Danilo; Petkovic-Curcin, Aleksandra; Sanz, Mariano (2013): Bone loss biomarkers associated with peri-implantitis. A cross-sectional study. In: Clinical oral implants research 24 (10), S. 1110-1116. DOI: 10.1111/j.1600-0501.2012.02518.x.
Ramanauskaite, Ausra; Fretwurst, Tobias; Schwarz, Frank (2021): Efficacy of alternative or adjunctive measures to conventional non-surgical and surgical treatment of peri-implant mucositis and peri-implantitis: a systematic review and meta-analysis. In: International journal of implant dentistry 7 (1), S. 112. DOI: 10.1186/s40729-021-00388-x.
Renvert, Stefan; Roos-Jansåker, Anne-Marie; Lindahl, Christel; Renvert, Helena; Rutger Persson, G. (2007): Infection at titanium implants with or without a clinical diagnosis of inflammation. In: Clinical oral implants research 18 (4), S. 509-516. DOI: 10.1111/j.1600-0501.2007.01378.x.
Ritchie, Matthew E.; Phipson, Belinda; Di Wu; Hu, Yifang; Law, Charity W.; Shi, Wei; Smyth, Gordon K. (2015): limma powers differential expression analyses for RNA-sequencing and microarray studies. In: Nucleic acids research 43 (7), e47. DOI: 10.1093/nar/gkv007.
Rizal, Muhammad Ihsan; Soeroso, Yuniarti; Sulijaya, Benso; Assiddiq, Bobby F.; Bachtiar, Endang W.; Bachtiar, Boy M. (2020): Proteomics approach for biomarkers and diagnosis of periodontitis: systematic review. In: Heliyon 6 (6), e04022. DOI: 10.1016/j.heliyon.2020.e04022.
Sahrmann, Philipp; Gilli, Fabienne; Wiedemeier, Daniel B.; Attin, Thomas; Schmidlin, Patrick R.; Karygianni, Lamprini (2020): The Microbiome of Peri-Implantitis: A Systematic Review and Meta-Analysis. In: Microorganisms 8 (5). DOI: 10.3390/microorganisms8050661.
Salazar, Manuela Gesell; Jehmlich, Nico; Murr, Annette; Dhople, Vishnu M.; Holtfreter, Birte; Hammer, Elke et al. (2013): Identification of periodontitis associated changes in the proteome of whole human saliva by mass spectrometric analysis. In: Journal of clinical periodontology 40 (9), S. 825-832. DOI: 10.1111/jcpe.12130.
Schäringer, Katja; Maxeiner, Sebastian; Schalla, Carmen; Rütten, Stephan; Zenke, Martin; Sechi, Antonio (2021): LSP1-myosin1e bimolecular complex regulates focal adhesion dynamics and cell migration. In: FASEB journal : official publication of the Federation of American Societies for Experimental Biology 35 (2), e21268. DOI: 10.1096/fj.202000740RR.
Schwarz, Frank (2016): AWMF-Leitlinie "Periimplantäre Infektionen an zahnimplantaten, Behandlung" 2016.
Shin, Myung-Seop; Kim, Yun-Gon; Shin, Yoo Jin; Ko, Byoung Joon; Kim, Sungtae; Kim, Hyun-Duck (2019): Deep sequencing salivary proteins for periodontitis using proteomics. In: Clinical oral investigations, S. 3571-3580. DOI: 10.1007/s00784-018-2779-1.
Sorsa, Timo; Gieselmann, Dirk; Arweiler, Nicole B.; Hernández, Marcela (2017): A quantitative point-of-care test for periodontal and dental peri-implant diseases. In: Nature reviews. Disease primers 3, S. 17069. DOI: 10.1038/nrdp.2017.69.
Sorsa, Timo; Gursoy, Ulvi K.; Nwhator, Solomon; Hernandez, Marcela; Tervahartiala, Taina; Leppilahti, Jussi et al. (2016): Analysis of matrix metalloproteinases, especially MMP-8, in gingival creviclular fluid, mouthrinse and saliva for monitoring periodontal diseases. In: Periodontology 2000 70 (1), S. 142-163. DOI: 10.1111/prd.12101.
Tholander, Fredrik; Muroya, Ayumo; Roques, Bernard-Pierre; Fournié-Zaluski, Marie-Claude; Thunnissen, Marjolein M. G. M.; Haeggström, Jesper Z. (2008): Structure-based dissection of the active site chemistry of leukotriene A4 hydrolase: implications for M1 aminopeptidases and inhibitor design. In: Chemistry & biology 15 (9), S. 920-929. DOI: 10.1016/j.chembiol. 2008.07.018.
Wang, Chin-Wei; Hao, Yuning; Di Gianfilippo, Riccardo; Sugai, James; Li, Jiaqian; Gong, Wang et al. (2021): Machine learning-assisted immune profiling stratifies peri-implantitis patients with unique microbial colonization and clinical outcomes. In: Theranostics 11 (14), S. 6703-6716. DOI: 10.7150/thno.57775.
Wang, Hom-Lay; Garaicoa-Pazmino, Carlos; Collins, Amy; Ong, Hwen-Sei; Chudri, Rini; Giannobile, William V. (2016): Protein biomarkers and microbial profiles in peri-implantitis. In: Clinical oral implants research 27 (9), S. 1129-1136. DOI: 10.1111/clr.12708.
Wasiluk, K. R.; Skubitz, K. M.; Gray, B. H. (1991): Comparison of granule proteins from human polymorphonuclear leukocytes which are bactericidal toward Pseudomonas aeruginosa. In: Infection and immunity 59 (11), S. 4193-4200. DOI: 10.1128/iai.59.11.4193-4200.1991.

## Claims

1. A method for detecting peri-implantitis (PI) in a mammalian subject having a tooth implant, comprising detecting, and quantifying at least one protein in a sulcus fluid sample obtained from said subject that is enriched or depleted in the sample when compared to a sulcus fluid sample i) obtained from a healthy implant (I), ii) a healthy tooth (T), iii) an earlier sulcus fluid sample taken from said subject, and/or iv) to a control sample, wherein an enrichment of the protein Immunoglobulin heavy variable 2-70D (IGHV70D), optionally together with an enrichment of at least one protein selected from the group consisting of, ARPC5, XRP2, DYSF, FLOT1, APOM, HGPRT, CA2, HBD, LRG, PRBP, CAT, CD18, SERPING1, MPO, HC-II, PYGL, GPI, BPGM, ANX6, RNASE2, ITGAM, G6PD, ANX3, ALADH, NQO2, BPI, ITGB5, AZU1, GGH, LTA4H, MIG9, HMG-2, STOM, GCA, TKT, DDT, PRDX3, SRI, CORO1A, LSP-1, CHI3L1, TALDO1, MNDA, CAMP, ARHGDIB, HK3, HBA1, BASP1, BST1, MTO, FLOT2, PLBD1, CNN2, SEC11A, GAPR-1, ADSF, APMAP, and PADI2
and/or optionally together with a depletion of at least one protein selected from the group consisting of COPE, NARS1, ATP5MG, EMAP-2, HLA-A, CTSD, ANX2, CAPNS1, CTSB, HSP90AA1, TACSTD2, TXN, COX4I1, PLS3, EZR, CBR1, RPL7, PEBP-1, S100A11, MDH1, ALDH9A1, NAPA, SLC25A3, PSMD2, PCN, TKFC, NAP-2, ACO2, ENOPH1, JPT1, and PSME2 detects a PI in said subject.

2. The method for detecting peri-implantitis (PI) in a mammalian subject having a tooth implant according to claim 1, wherein at least one protein in the sulcus fluid sample selected from the group consisting of FLOT1, APOM, CD18, MPO, ITGAM, BPI, ITGB5, FLOT2, PLBD1, ADSF, and APMAP, and/or CTSD is secreted or shedded.

3. The method for detecting peri-implantitis (PI) in a mammalian subject having a tooth implant according to claim 1 or 2, wherein the significance of the at least one protein has a moderated p-value of < 0.05.

4. The method for detecting peri-implantitis (PI) in a mammalian subject having a tooth implant according to any one of claims 1 to 3, wherein the sulcus fluid sample was obtained from said subject using sterile paper strips such as, for example, are endodontic paper points or periopapers.

5. The method for detecting peri-implantitis (PI) in a mammalian subject having a tooth implant according to any one of claims 1 to 4, wherein the detection and quantification of the at least one protein in the sample comprises a method selected from at least one of ELISA, proteolysis, bicinchoninic acid assay, and mass spectrometry.

6. The method for detecting peri-implantitis (PI) in a mammalian subject having a tooth implant according to any one of claims 1 to 5, further comprising detecting endogenous proteolysis in the sample, wherein a higher endogenous proteolytic activity as detected when compared to a non-PI sample, a T sample, an I sample and/or a control sample indicates a PI sample.

7. The method for detecting peri-implantitis (PI) in a mammalian subject having a tooth implant according claim 6, wherein detecting a higher endogenous proteolytic activity comprises detecting of proportional intensities of semi-specific peptides.

8. The method for detecting peri-implantitis (PI) in a mammalian subject having a tooth implant according to any one of claims 1 to 7, further comprising detecting and quantifying bacterial proteins in the sample, wherein a higher amount as detected when compared to a non-PI sample, a T sample, an I sample and/or a control sample indicates a PI sample.

9. The method for detecting peri-implantitis (PI) in a mammalian subject having a tooth implant according to any one of claims 1 to 8, further comprising detecting the missingness of bacterial proteins in the sample, wherein a lower missingness as detected when compared to a non-PI sample, a T sample, an I sample and/or a control sample indicates a PI sample.

10. The method for detecting peri-implantitis (PI) in a mammalian subject having a tooth implant according to any one of claims 1 to 9, wherein the mammalian subject is selected from a cat, dog, mouse, rat, horse, sheep, goat, monkey, cow, or human, preferably a human.

11. A method for diagnosing the status of peri-implantitis (PI) in a mammalian subject having a tooth implant, comprising performing the method according to any one of claims 1 to 10, and diagnosing an exacerbated state of the PI if the at least one protein in the sulcus fluid sample obtained from said subject is further enriched or further depleted in the sample when compared to an earlier sulcus fluid sample obtained from the subject.

12. A method for identifying a compound that is active against PI, comprising the steps of performing the method according to any one of claims 1 to 10 on a sulcus fluid sample obtained from a subject suffering from PI that has been administered with a candidate compound, wherein a lesser enrichment and/or a lesser depletion of at least one protein in the sulcus fluid sample in the presence of said candidate compound, when compared to the absence of said candidate compound identifies a compound that is active against PI.

13. The method for identifying a compound that is active against PI according to claim 12, wherein the candidate compound is selected from the group consisting of a chemical molecule, a molecule selected from a library of small organic molecules, a molecule selected from a combinatory library, a cell extract, in particular a plant cell extract, a small molecular drug, a protein, a protein fragment, a molecule selected from a peptide library, and an antibody or fragment thereof.

14. A method for monitoring of a treatment or prophylaxis against PI in a mammalian subject in need thereof, comprising a) performing the method according to any one of claims 1 to 10 on a biological sample obtained from a subject that has been administered with a compound or pharmaceutical composition that is active against PI, and b) comparing the enrichment and/or depletion of at least one protein in the sulcus fluid sample as detected with the enrichment and/or depletion of the at least one protein in an earlier sulcus fluid sample taken from said subject, and/or to a control sample.

## Patentansprüche

1. Verfahren zum Nachweis von Periimplantitis (PI) in einem Säugetier-Subjekt, das ein Zahnimplantat aufweist, umfassend ein Nachweisen und Quantifizieren von mindestens einem Protein in einer von dem Subjekt erhaltenen Sulcusflüssigkeitsprobe, wobei das Protein in der Probe angereichert oder verringert ist, wenn mit einer Sulcusflüssigkeitsprobe verglichen, die i) von einem gesunden Implantat erhalten wurde (I), ii) einem gesunden Zahn (T), iii) einer früheren Sulcusflüssigkeitsprobe, die von dem Subjekt entnommen wurde, und/oder iv) einer Kontrollprobe, wobei eine Anreicherung des Proteins Immnunglobulin schwere variable 2-70D (IGHV70D), gegebenenfalls zusammen mit einer Anreicherung von mindestens einem Protein ausgewählt aus der Gruppe bestehend aus ARPC5, XRP2, DYSF, FLOT1, APOM, HGPRT, CA2, HBD, LRG, PRBP, CAT, CD18, SERPING1, MPO, HC-II, PYGL, GPI, BPGM, ANX6, RNASE2, ITGAM, G6PD, ANX3, ALADH, NQO2, BPI, ITGB5, AZU1, GGH, LTA4H, MIG9, HMG-2, STOM, GCA, TKT, DDT, PRDX3, SRI, CORO1A, LSP-1, CHI3L1, TALDOl, MNDA, CAMP, ARHGDIB, HK3, HBA1, BASP1, BST1, MTO, FLOT2, PLBD1, CNN2, SEC11A, GAPR-1, ADSF, APMAP und PADI2
und/oder gegebenenfalls zusammen mit einer Verringerung von mindestens einem Protein ausgewählt aus der Gruppe bestehend aus COPE, NARS1, ATP5MG, EMAP-2, HLA-A, CTSD, ANX2, CAPNS1, CTSB, HSP90AA1, TACSTD2, TXN, COX4I1, PLS3, EZR, CBR1, RPL7, PEBP-1, S100A11, MDH1, ALDH9A1, NAPA, SLC25A3, PSMD2, PCN, TKFC, NAP-2, ACO2, ENOPH1, JPT1 und PSME2 eine PI in dem Subjekt nachweist.

2. Verfahren zum Nachweis von Periimplantitis (PI) in einem Säugetier-Subjekt, das ein Zahnimplantat aufweist nach Anspruch 1, wobei das mindestens eine Protein in der Sulcusflüssigkeitsprobe ausgewählt aus der Gruppe bestehend aus FLOT1, APOM, CD18, MPO, ITGAM, BPI, ITGB5, FLOT2, PLBD1, ADSF, and APMAP und/oder CTSD sekretiert oder abgespalten wird.

3. Verfahren zum Nachweis von Periimplantitis (PI) in einem Säugetier-Subjekt, das ein Zahnimplantat aufweist nach Anspruch 1 oder 2, wobei die Signifikanz des mindestens einen Proteins einen moderierten p-Wert von < 0,05 aufweist.

4. Verfahren zum Nachweis von Periimplantitis (PI) in einem Säugetier-Subjekt, das ein Zahnimplantat aufweist nach einem der Ansprüche 1 bis 3, wobei die Sulcusflüssigkeitsprobe von dem Subjekt unter der Verwendung von sterilen Papierstreifen, wie zum Beispiel endodontischen Papierspitzen oder perioperativem Papier erhalten wurde.

5. Verfahren zum Nachweis von Periimplantitis (PI) in einem Säugetier-Subjekt, das ein Zahnimplantat aufweist nach einem der Ansprüche 1 bis 4, wobei der Nachweis und die Quantifizierung des mindestens einen Proteins in der Probe ein Verfahren ausgewählt aus mindestens einem von ELISA, Proteolyse, Bicinchoninsäure Assay und Massenspektrometrie umfasst.

6. Verfahren zum Nachweis von Periimplantitis (PI) in einem Säugetier-Subjekt, das ein Zahnimplantat aufweist nach einem der Ansprüche 1 bis 5, weiter umfassend ein Nachweisen von endogener Proteolyse in der Probe, wobei eine höhere endogene proteolytische Aktivität wie nachgewiesen, wenn mit einer nicht-PI Probe, einer T Probe, einer I Probe und/oder einer Kontrollprobe verglichen, eine PI Probe anzeigt.

7. Verfahren zum Nachweis von Periimplantitis (PI) in einem Säugetier-Subjekt, das ein Zahnimplantat aufweist nach Anspruch 6, wobei der Nachweis einer höheren endogenen proteolytischen Aktivität ein Nachweisen von proportionalen Intensitäten von semispezifischen Peptiden umfasst.

8. Verfahren zum Nachweis von Periimplantitis (PI) in einem Säugetier-Subjekt, das ein Zahnimplantat aufweist nach einem der Ansprüche 1 bis 7, weiter umfassend ein Nachweisen und die Quantifizierung von bakteriellen Proteinen in der Probe, wobei eine höhere Menge wie nachgewiesen, wenn mit einer nicht-PI Probe, einer T Probe, einer I Probe und/oder einer Kontrollprobe verglichen, eine PI Probe anzeigt.

9. Verfahren zum Nachweis von Periimplantitis (PI) in einem Säugetier-Subjekt, das ein Zahnimplantat aufweist nach einem der Ansprüche 1 bis 8, weiter umfassend ein Nachweisen der Missingness von bakteriellen Proteinen in der Probe, wobei einer geringere Missingness wie nachgewiesen, wenn mit einer nicht-PI Probe, einer T Probe, einer I Probe und/oder einer Kontrollprobe verglichen, eine PI Probe anzeigt.

10. Verfahren zum Nachweis von Periimplantitis (PI) in einem Säugetier-Subjekt, das ein Zahnimplantat aufweist nach einem der Ansprüche 1 bis 9, wobei das Säugetier-Subjekt ausgewählt ist von einer Katze, einem Hund, einer Maus, einer Ratte, einem Pferd, einem Schaf, einer Ziege, einem Affen, einer Kuh oder einem Menschen, bevorzugt einem Menschen.

11. Verfahren zur Diagnose der Status von Periimplantitis (PI) in einem Säugetier-Subjekt, das ein Zahnimplantat aufweist, umfassend ein Durchführen des Verfahrens nach einem der Ansprüche 1 bis 10 und Diagnostizieren eines verschlimmerten Zustands der PI, wenn das mindestens eine Protein in der Sulcusflüssigkeitsprobe wie erhalten von dem Subjekt in der Probe weiter angereichert ist oder verringert ist, wenn verglichen mit einer früheren von dem Subjekt erhaltenen Sulcusflüssigkeitsprobe.

12. Verfahren zur Identifizierung einer Verbindung die gegen PI wirksam ist, umfassend die Schritte von Durchführen des Verfahrens nach einem der Ansprüche 1 bis 10 an einer Sulcusflüssigkeitsprobe wie erhalten von dem Subjekt, das an PI leidet, an das eine Kandidatenverbindung verabreicht wurde, wobei eine geringere Anreichung und/oder eine geringere Abreicherung des mindestens einen Proteins in der Sulcusflüssigkeitsprobe in der Anwesenheit der Kandidatenverbindung, wenn verglichen mit der Abwesenheit der Kandidatenverbindung eine Verbindung identifiziert, die gegen PI aktiv ist.

13. Verfahren zur Identifizierung einer Verbindung die gegen PI wirksam ist nach Anspruch 12, wobei die Kandidatenverbindung ausgewählt ist aus der Gruppe bestehend aus einem chemischen Molekül, einem Molekül ausgewählt aus einer Bibliothek von kleinen organischen Molekülen, einem Molekül ausgewählt aus einer kombinatorischen Bibliothek, einem Zellextrakt, insbesondere einem Pflanzenzellextrakt, einem Wirkstoff mit kleinem Molekulargewicht, einem Protein, einem Proteinfragment, einem Molekül ausgewählt aus einer Peptidbibliothek und einem Antikörper oder Fragment davon.

14. Verfahren zur Überwachung einer Behandlung oder Prophylaxe gegen PI in einem Säugetier-Subjekt, der dieser Behandlung bedarf, umfassend a) Durchführen des Verfahrens nach einem der Ansprüche 1 bis 10 an einer biologischen Probe wie erhalten von einem Subjekt, dem eine Verbindung oder pharmazeutischen Zusammensetzung die gegen PI aktiv ist verabreicht wurde, und b) Vergleichen der Anreicherung und/oder einer Depletion des mindestens einen Proteins in der Sulcusflüssigkeitsprobe wie nachgewiesen mit der Anreicherung und/oder einer Depletion des mindestens einen Proteins in einer früheren Sulcusflüssigkeitsprobe die von dem Subjekt genommen wurde und/oder mit einer Kontrollprobe.

## Revendications

1. Procédé de détection de la péri-implantite (PI) chez un sujet mammifère ayant un implant dentaire, comprenant la détection et la quantification d'au moins une protéine dans un échantillon de liquide sulculaire obtenu à partir dudit sujet, qui est enrichi ou appauvri dans l'échantillon par rapport à un échantillon de liquide sulculaire i) obtenu à partir d'un implant sain (1), ii) d'une dent saine (T), iii) d'un échantillon de liquide sulculaire prélevé antérieurement sur ledit sujet, et/ou iv) à un échantillon témoin, dans lequel un enrichissement de la protéine Immunoglobuline lourde variable 2-70D (IGHV70D), éventuellement associé à un enrichissement d'au moins une protéine choisie dans le groupe constitué par ARPC5, XRP2, DYSF, FLOT1, APOM, HGPRT, CA2, HBD, LRG, PRBP, CAT, CD18, SERPING1, MPO, HC-II, PYGL, GPI, BPGM, ANX6, RNASE2, ITGAM, G6PD, ANX3, ALADH, NQO2, BPI, ITGB5, AZU1, GGH, LTA4H, MIG9, HMG-2, STOM, GCA, TKT, DDT, PRDX3, SRI, CORO1A, LSP-1, CHI3L1, TALDO1, MNDA, CAMP, ARHGDIB, HK3, HBA1, BASP1, BST1, MTO, FLOT2, PLBD1, CNN2, SEC11A, GAPR-1, ADSF, APMAP, et PADI2
et/ou éventuellement associé à un appauvrissement en au moins une protéine choisie dans le groupe constitué par COPE, NARS1, ATP5MG, EMAP-2, HLA-A, CTSD, ANX2, CAPNS1, CTSB, HSP90AA1, TACSTD2, TXN, COX4I1, PLS3, EZR, CBR1, RPL7, PEBP-1, S100A11, MDH1, ALDH9A1, NAPA, SLC25A3, PSMD2, PCN, TKFC, NAP-2, ACC2, ENOPH1, JPT1 et PSME2 détecte une PI chez ledit sujet.

2. Procédé de détection de la péri-implantite (PI) chez un sujet mammifère ayant un implant dentaire selon la revendication 1, dans lequel au moins une protéine dans l'échantillon de liquide sulculaire choisie dans le groupe constitué par FLOT1, APOM, CD18, MPO, ITGAM, BPI, ITGB5, FLOT2, PLBD1, ADSF et APMAP, et/ou CTSD est sécrétée ou libérée.

3. Procédé de détection de la péri-implantite (PI) chez un sujet mammifère ayant un implant dentaire selon la revendication 1 ou 2, dans lequel la signification de l'au moins une protéine a une valeur p modérée < 0,05.

4. Procédé de détection de la péri-implantite (PI) chez un sujet mammifère ayant un implant dentaire selon l'une quelconque des revendications 1 à 3, dans lequel l'échantillon de liquide sulculaire a été obtenu à partir dudit sujet à l'aide de bandes de papier stériles telles que, par exemple, des pointes de papier endodontique ou des papiers périopératoires.

5. Procédé de détection de la péri-implantite (PI) chez un sujet mammifère ayant un implant dentaire selon l'une quelconque des revendications 1 à 4, dans lequel la détection et la quantification de l'au moins une protéine dans l'échantillon comprend un procédé choisi parmi au moins l'un des procédés suivants : ELISA, protéolyse, test à l'acide bicinchoninique, et spectrométrie de masse.

6. Procédé de détection de la péri-implantite (PI) chez un sujet mammifère ayant un implant dentaire selon l'une quelconque des revendications 1 à 5, comprenant en outre la détection de la protéolyse endogène dans l'échantillon, dans lequel une activité protéolytique endogène plus élevée telle que détectée par rapport à un échantillon non-PI, un échantillon T, un échantillon I et/ou un échantillon témoin indique un échantillon PI.

7. Procédé de détection de la péri-implantite (PI) chez un sujet mammifère ayant un implant dentaire selon la revendication 6, dans lequel la détection d'une activité protéolytique endogène plus élevée comprend la détection d'intensités proportionnelles de peptides semi-spécifiques.

8. Procédé de détection de la péri-implantite (PI) chez un sujet mammifère ayant un implant dentaire selon l'une quelconque des revendications 1 à 7, comprenant en outre la détection et la quantification de protéines bactériennes dans l'échantillon, dans lequel une quantité plus élevée telle que détectée par rapport à un échantillon non-PI, un échantillon T, un échantillon I et/ou un échantillon témoin indique un échantillon PI.

9. Procédé de détection de la péri-implantite (PI) chez un sujet mammifère ayant un implant dentaire selon l'une quelconque des revendications 1 à 8, comprenant en outre la détection de l'absence de protéines bactériennes dans l'échantillon, dans lequel une absence plus faible telle que détectée par rapport à un échantillon non-PI, un échantillon T, un échantillon I et/ou un échantillon témoin indique un échantillon PI.

10. Procédé de détection de la péri-implantite (PI) chez un sujet mammifère ayant un implant dentaire selon l'une quelconque des revendications 1 à 9, dans lequel le sujet mammifère est choisi parmi un chat, un chien, une souris, un rat, un cheval, un mouton, une chèvre, un singe, une vache ou un être humain, de préférence un être humain.

11. Procédé de diagnostic de l'état de péri-implantite (PI) chez un sujet mammifère ayant un implant dentaire, comprenant la mise en œuvre du procédé selon l'une quelconque des revendications 1 à 10, et le diagnostic d'un état exacerbé de la PI si l'au moins une protéine dans l'échantillon de liquide sulculaire obtenu à partir dudit sujet est davantage enrichie ou davantage appauvrie dans l'échantillon par rapport à un échantillon de liquide sulculaire antérieur obtenu à partir du sujet.

12. Procédé d'identification d'un composé actif contre la PI, comprenant les étapes consistant à mettre en œuvre le procédé selon l'une quelconque des revendications 1 à 10 sur un échantillon de liquide sulculaire obtenu à partir d'un sujet souffrant de PI auquel a été administré un composé candidat, dans lequel un enrichissement moindre et/ou un appauvrissement moindre d'au moins une protéine dans l'échantillon de liquide sulculaire en présence dudit composé candidat, par rapport à l'absence dudit composé candidat, identifie un composé actif contre la PI.

13. Procédé d'identification d'un composé actif contre la PI selon la revendication 12, dans lequel le composé candidat est choisi parmi le groupe constitué d'une molécule chimique, d'une molécule choisie dans une bibliothèque de petites molécules organiques, d'une molécule choisie dans une bibliothèque combinatoire, d'un extrait cellulaire, en particulier d'un extrait cellulaire végétal, d'un médicament à petites molécules, d'une protéine, d'un fragment de protéine, d'une molécule choisie dans une bibliothèque de peptides, et d'un anticorps ou d'un fragment de celui-ci.

14. Procédé de surveillance d'un traitement ou d'une prophylaxie contre la PI chez un sujet mammifère qui en a besoin, comprenant a) la mise en œuvre du procédé selon l'une quelconque des revendications 1 à 10 sur un échantillon biologique obtenu à partir d'un sujet auquel a été administré un composé ou une composition pharmaceutique actif contre la PI**,** et b) la comparaison de l'enrichissement et/ou de l'appauvrissement d'au moins une protéine dans l'échantillon de liquide sulculaire tel que détecté avec l'enrichissement et/ou l'appauvrissement de ladite au moins une protéine dans un échantillon de liquide sulculaire antérieur obtenu à partir du sujet, et/ou un échantillon témoin.
